Europäisches Patentamt

European Patent Office

Office européen des brevets

(11) Publication number: **0 088 622 B1**

# EUROPEAN PATENT SPECIFICATION

(45) Date of publication of patent specification: **14.06.95**

(51) Int. Cl.⁶: **C12N 15/20**, C12N 5/10, C12N 1/21, C12P 21/02, A61K 38/21, //C12R1/19

(21) Application number: **83301208.1**

(22) Date of filing: **07.03.83**

(54) **Animal interferons, processes involved in their production, compositions containing them, DNA sequences coding therefor and expression vehicles containing such sequences and cells transformed thereby.**

(30) Priority: **08.03.82 US 355298**
        **01.11.82 US 438128**

(43) Date of publication of application:
**14.09.83 Bulletin 83/37**

(45) Publication of the grant of the patent:
**14.06.95 Bulletin 95/24**

(84) Designated Contracting States:
**AT BE CH DE FR GB IT LI LU NL SE**

(56) References cited:
**EP-A- 0 042 246**
**WO-A-80/02375**
**US-A- 4 262 090**

**CHEMICAL ABSTRACTS, vol.92, no. 3, January 21, 1980, page 517, abstract 20424u, Columbus, Ohio, US; T.G. ORLOVA et al.**

**NULEIC ACIDS RESEARCH, vol 10, no. 10, 1982, IRL Press Ltd, London, GB; D.SKUP et al., pages 3069-3084**

(73) Proprietor: **GENENTECH, INC.**
**460 Point San Bruno Boulevard**
**South San Francisco**
**California 94080 (US)**

(72) Inventor: **Capon, Daniel Jeffrey**
**3914 18th Street**
**San Francisco**
**California 94114 (US)**
Inventor: **Goeddel, David VanNorman**
**2115 Forestview**
**Hillsborough**
**California 94010 (US)**

(74) Representative: **Armitage, Ian Michael et al**
**MEWBURN ELLIS**
**York House**
**23 Kingsway**
**London WC2B 6HP (GB)**

NUCLEIC ACIDS RESEARCH, vol. 11, no. 3, 1983, IRL Press Ltd, Oxford, GB; G.D. SHAW et al., pages 555-573

**Description**

Field of the Invention

The present invention relates generally to the field of recombinant DNA technology, to means and methods utilizing such technology in the discovery of a broad class of animal interferons and to the production thereof and to the various products of such production and their uses.

More particularly the present invention relates to the isolation and identification of DNA sequences encoding animal interferons and to the construction of recombinant DNA expression vehicles containing such DNA sequences operably linked to expression-effecting promoter sequences and to the expression vehicles so constructed. In another aspect, the present invention relates to host culture systems, such as various microorganism and vertebrate cell cultures transformed with such expression vehicles and thus directed in the expression of the DNA sequences referred to above. In yet other aspects, this invention relates to the means and methods of converting the novel end products of such expression to entities, such as pharmaceutical compositions, useful for the prophylactic or therapeutic treatment of animals. In addition, this invention relates to various processes useful for producing said DNA sequences, expression vehicles, host culture systems and end products and entities thereof and to specific and associated embodiments thereof.

The present invention arises in part from the discovery of the DNA sequence and deduced amino acid sequence encoding a series of bovine alpha interferons, including the 3'- and 5'-flanking sequences thereof facilitating their in vitro linkage into expression vehicles. These discoveries, in turn, enable the development of the means and methods for producing, via recombinant DNA technology, sufficient amounts of animal interferons, so as to enable, in turn, the determination of their biochemical properties and bioactivity, making possible their efficient production for commercial/biological exploitation.

The publications and other materials hereof used to illuminate the background of the invention, and in particular cases, to provide additional details respecting its practice are hereby incorporated herein by this reference, and for convenience, are numerically referenced by the following text and respectively grouped in the appended bibliography.

Background of the Invention

A. Animal Interferons

Interferon components have been isolated from tissues of various phylogenetic species lower than human (1,2,3). Activity studies conducted with these interferons have demonstrated varying degrees of antiviral effects in the requisite host animal (3,4,5,6). It also has been demonstrated that these interferons are not always species specific. For example, preparations of bovine interferons isolated from tissues, had antiviral activity on monkey and human cells (7). Likewise, human interferons have been found active in various cells of phylogenetically lower species (see 7).

This species interactivity is doubtless due to a high degree of homologous conservation, both in amino acid composition and sequence, amongst the interferons. However, until now, this explanation remained theoretical because the amounts and purities of animal interferons that have been obtainable were insufficient to carry out unambiguous experiments on the characterization and biological properties of the purified components versus several of their human counterparts (8,9,10,11,12).

In any event, despite these low amounts and purities, a causal connection between interferon and anti-viral activity in the requisite animal host has been established. Thus, the production of animal interferons in high yields and purities would be very desirable in order to initiate and successfully conduct animal bioassay experiments leading toward commercial exploitation in the treatment of animals for viral infections and malignant and immunosuppressed or immunodeficient conditions. In addition, the production of isolated animal interferon species would enable their characterization, both physical and bioactive, and thus provide a basis for categorization and consequential comparative studies with counterpart human interferon species (see 8 to 20).

The studies done with animal interferons, until the present invention, being restricted to the use of rather crude preparations, due to their very low availability, nevertheless suggest very important biological functions. Not only have the class of animal interferons a potent associated therapeutic antiviral activity, but also potential as a prophylactic adjunct with vaccine and/or antibiotic treatment, clearly pointing to very promising clinical and commercial candidates.

It was perceived that the application of recombinant DNA technology would be a most effective way of providing the requisite larger quantities of animal interferons necessary to achieve clinical and commercial exploitation. Whether or not the materials so produced would include glycosylation which is considered characteristic of native derived material, they would probably exhibit bioactivity admitting of their use clinically in the treatment of a wide range of viral, neoplastic, and immunosuppressed conditions or diseases in animals.

B. Recombinant DNA Technology

Recombinant DNA technology has reached the age of some sophistication. Molecular biologists are able to recombine various DNA sequences with some facility, creating new DNA entities capable of producing copious amounts of exogenous protein product in transformed microbes. The general means and methods are in hand for the in vitro ligation of various blunt ended or "sticky" ended fragments of DNA, producing potent expression vehicles useful in transforming particular organisms, thus directing their efficient synthesis of desired exogenous product. However, on an individual product basis, the pathway remains somewhat tortuous and the science has not advanced to a stage where regular predictions of success can be made. Indeed, those who portend successful results without the underlying experimental basis, do so with considerable risk of inoperability.

The plasmid, an extrachromosomal loop of double-stranded DNA found in bacteria and other microbes, oftentimes in multiple copies per cell, remains a basic element of recombinant DNA technology. Included in the information encoded in the plasmid DNA is that required to reproduce the plasmid in daughter cells (i.e., an origin of replication) and ordinarily, one or more phenotypic selection characteristics such as, in the case of bacteria, resistance to antibiotics, which permit clones of the host cell containing the plasmid of interest to be recognized and preferentially grown in selective media. The utility of plasmids lies in the fact that they can be specifically cleaved by one or another restriction endonuclease or "restriction enzyme", each of which recognizes a different site on the plasmid DNA. Thereafter heterologous genes or gene fragments may be inserted into the plasmid by endwise joining at the cleavage site or at reconstructed ends adjacent to the cleavage site. Thus formed are so-called replicable expression vehicles. DNA recombination is performed outside the cell, but the resulting "recombinant" replicable expression vehicle, or plasmid, can be introduced into cells by a process known as transformation and large quantities of the recombinant vehicle obtained by growing the transformant. Moreover, where the gene is properly inserted with reference to portions of the plasmid which govern the transcription and translation of the encoded DNA message, the resulting expression vehicle can be used to actually produce the polypeptide sequence for which the inserted gene codes, a process referred to as expression.

Expression is initiated in a region known as the promoter which is recognized by and bound by RNA polymerase. In the transcription phase of expression, the DNA unwinds, exposing it as a template for initiated synthesis of messenger RNA from the DNA sequence. The messenger RNA is, in turn, translated into a polypeptide having the amino acid sequence encoded by the mRNA. Each amino acid is encoded by a nucleotide triplet or "codon" which collectively make up the "structural gene", i.e. that part which encodes the amino acid sequence of the expressed polypeptide product. Translation is initiated at a "start" signal (ordinarily ATG, which in the resulting messenger RNA becomes AUG). So-called stop codons define the end of translation and, hence, of production of further amino acid units. The resulting product may be obtained by lysing, if necessary, the host cell, in microbial systems, and recovering the product by appropriate purification from other proteins.

In practice, the use of recombinant DNA technology can express entirely heterologous polypeptides--so-called direct expression--or alternatively may express a heterologous polypeptide fused to a portion of the amino acid sequence of a homologous polypeptide. In the latter cases, the intended bioactive product is sometimes rendered bioinactive within the fused, homologous/heterologous polypeptide until it is cleaved in an extracellular environment (21, 22).

C. Cell Culture Technology

The art of cell or tissue cultures for studying genetics and cell physiology is well established. Means and methods are in hand for maintaining permanent cell lines, prepared by successive serial transfers from isolate normal cells. For use in research, such cell lines are maintained on a solid support in liquid medium, or by growth in suspension containing support nutriments. Scale-up for large preparations seems to pose only mechanical problems (See generally 23,24).

4

## Summary of the Invention

The present invention is based upon the discovery that recombinant DNA technology can be used to successfully produce animal interferons, and each of them, preferably in direct form, and in amounts sufficient to initiate and conduct clinical testing as prerequisites to market approval. The product is suitable for use, in all of its forms, in the prophylactic or therapeutic treatment of animals, notably for viral infections and malignant and immunosuppressed or immunodeficient conditions. Its forms include various possible oligomeric forms which may include associated glycosylation as well as allelic variations of individual members or family units. The products are produced by genetically engineered microorganisms or cell culture systems. Thus, the potential now exists to prepare and isolate animal interferons in a more efficient manner than has been possible. One significant factor of the present invention, in its most preferred embodiment, is the accomplishment of genetically directing a microorganism or cell culture to produce a representative animal interferon, bovine interferon, in isolatable amounts, produced by the host cell in mature form.

The present invention provides a method for identifying DNA encoding non-human animal interferons. The present invention is further directed to replicable DNA expression vehicles harboring such DNA in expressible form. Further, the present invention is directed to microorganism strains or cell cultures transformed with the expression vehicles described above and to fermentation media comprising such transformed strains or cultures, capable of producing animal interferons.

In certain host systems, vectors can be devised to produce the desired animal interferon, secreted from the host cell in mature form. The interferon containing the signal sequence derived from the 5'-flanking region of the gene proper is believed to be transported to the cellular wall of the host organisms where, aiding in such transport, the signal portion is cleaved during the secretion process of the mature interferon product. This embodiment enables the isolation and purification of the intended mature interferon without resort to involved procedures designed to eliminate contaminants of intracellular host protein or cellular debris.

In addition, this invention is specifically directed to the preparation of a bovine interferon representative of the class of animal interferons embraced herein, produced by direct expression in mature form.

Reference herein to the expression "mature animal interferon" connotes the microbial or cell culture production of animal interferon unaccompanied by the signal peptide or presequence peptide that immediately attends translation of the animal interferon mRNA. Mature animal interferon, according to the present invention, is thus provided, having methionine as its first amino acid (present by virtue of the ATG start signal codon insertion in front of the structural gene) or, where the methionine is intra- or extracellularly cleaved, having its normally first amino acid. Mature animal interferon can also be produced, in accordance herewith, together with a conjugated protein other than the conventional signal polypeptide, the conjugate being specifically cleavable in an intra- or extracellular environment (see 21). Finally, the mature animal interferon can be produced by direct expression without the necessity of cleaving away any extraneous, superfluous polypeptide. This is particularly important where a given host may not, or not efficiently, remove a signal peptide where the expression vehicle is designed to express the mature interferon together with its signal peptide. The thus produced mature interferon is recovered and purified to a level fitting it for use in the treatment of viral, malignant, and immunosuppressed or immunodeficient conditions.

Animal interferons hereof are those otherwise endogenous to the animal organism including, in nomenclature analogous to human interferons, animal alpha (leukocyte), beta (fibroblast) and gamma (immune) interferons. All three series have been identified in an animal model. Further, based upon the bovine example, the animal alpha series is composed of a family of proteins as in the human case; those investigated have a lower degree of homology to the corresponding human alpha interferons than either those animal interferons have amongst themselves or the human alpha interferons have amongst themselves. In addition, the bovine beta series is composed of a family of proteins, distinct from the human case. In addition, this invention provides interspecies and intrafamily hybrid interferons, by taking advantage of common restriction sites within the genes of the various animal interferons hereof and recombining corresponding portions, according to known methods (see 57).

In any event, the animal interferons described by this invention include those normally endogenous to animals of the avian, bovine, canine, equine, feline, hircine, ovine, piscine, and porcine families. In particular, the present invention relates to interferons of cloven-hooved animals such as cattle, sheep and goats. The bovine interferons provided by this invention find application as antiviral and antitumor agents in the respective host animal. For example, bovine interferons would find practical applications in treating respiratory complex in cattle, either in conjunction with (per se known) antibiotics as a therapeutic component or with vaccines as a prophylactic component. Class utility, demonstrated as described above, would extend to other bovine, and to goats, sheep, pigs, horses, dogs, cats, birds and fish. In horses, dogs, cats and birds, the antitumor effect of the corresponding interferons could be expected to be especially important commercially.

The following rationale, described with reference to bovine interferon as a representative of the class, may be employed for obtaining animal interferons hereof, in accordance with this invention:

1. Bovine tissues, for example bovine pancreas tissue, were reduced to frozen powder and treated to digest RNA and protein materials and provide, on precipitation, high molecular weight bovine DNA.

2. The high molecular weight DNA was partially digested for random cutting with respect to gene locus.

3. The resultant DNA fragments were size-fractionated giving from 15 to 20 kilo base pair fragments.

4. The resultant fragments of Step 3 were cloned using a λ Charon 30 phage vector.

5. The thus prepared vectors were packaged in vitro to infectious phage particles containing rDNA to provide a phage library. This was amplified by propagation on bacterial cells to about $10^6$ fold. The phage were plated to virtual confluence on a lawn of bacteria and screened for hybridization with a radioactive human interferon probe.

6. From the appropriate clones the corresponding DNA was isolated and restriction mapped and analyzed by Southern hybridization. Restriction fragments containing bovine interferon genes were subcloned into plasmid vehicles and then sequenced.

7. The sequenced DNA was then tailored in vitro for insertion into an appropriate expression vehicle which was used to transform an appropriate host cell which was, in turn, permitted to grow in a culture and to express the desired bovine interferon product.

8. Bovine interferon thus produced has 166 amino acids in its mature form, beginning with cysteine, and 23 in the presequence, and is very hydrophobic in character. Its monomeric molecular weight has been calculated at about 21,409. It displays characteristics similar to human leukocyte interferons (8,9,10,11) and has been found to be about 60 percent homologous to a human leukocyte interferon.

Description of Preferred Embodiments

A. Microorganisms/Cell Cultures

1. Bacterials Strains/Promoters

The work described herein was performed employing, inter alia, the microorganism E. coli K-12 strain 294 (end A, thi⁻, hsr⁻, ₖhsm⁺) (25). This strain has been deposited with the American Type Culture Collection, ATCC Accession No. 31446. However, various other microbial strains are useful, including known E. coli strains such as E. coli B, E. coli X 1776 (ATCC No. 31537) and E. coli W 3110 (F⁻, λ⁻, protrophic) (ATCC No. 27325), E. coli DP 50 SuPF (ATCC No. 39061, deposited March 5, 1982), E. coli JM83 (ATCC No. 39062, deposited March 5, 1982) or other microbial strains many of which are deposited and (potentially) available from recognized microorganism depository institutions, such as the American Type Culture Collection (ATCC)--cf. the ATCC catalogue listing (See also 26, 26a). These other microorganisms include, for example, Bacilli such as Bacillus subtilis and other enterobacteriaceae among which can be mentioned as examples Salmonella typhimurium and Serratia marcesans, utilizing plasmids that can replicate and express heterologous gene sequences therein.

As examples, the beta lactamase and lactose promoter systems have been advantageously used to initiate and sustain microbial production of heterologous polypeptides. Details relating to the make-up and construction of these promoter systems can be obtained by reference to (27) and (28). More recently, a system based upon the tryptophan operon, the so-called trp promoter system, has been developed. Details relating to the make-up and construction of this system have been published by Goeddel et al. (12) and Kleid et al. (29). Numerous other microbial promoters have been discovered and utilized and details concerning their nucleotide sequences, enabling a skilled worker to ligate them functionally within plasmid vectors, have been published -- see (30).

2. Yeast Strains/Yeast Promoters

The expression system hereof may also employ the plasmid YRp7 (31, 32, 33), which is capable of selection and replication in both E. coli and the yeast, Saccharomyces cerevisiae. For selection in yeast the plasmid contains the TRP1 gene (31, 32, 33) which complements (allows for growth in the absence of tryptophan) yeast containing mutations in this gene found on chromosome IV of yeast (34). One useful strain is strain RH218 (35) deposited at the American Type Culture Collection without restriction (ATCC No.

44076). However, it will be understood that any Saccharomyces cerevisiae strain containing a mutation which makes the cell trp1 should be an effective environment for expression of the plasmid containing the expression system. An example of another strain which could be used is pep4-1 (36). This tryptophan auxotroph strain also has a point mutation in TRP1 gene.

When placed on the 5' side of a non-yeast gene the 5'-flanking DNA sequence (promoter) from a yeast gene (for alcohol dehydrogenase 1) can promote the expression of a foreign gene in yeast when placed in a plasmid used to transform yeast. Besides a promoter, proper expression of a non-yeast gene in yeast requires a second yeast sequence placed at the 3'-end of the non-yeast gene on the plasmid so as to allow for proper transcription termination and polyadenylation in yeast. This promoter can be suitably employed in the present invention as well as others -- see infra. In the preferred embodiments, the 5'-flanking sequence of the yeast 3-phosphoglycerate kinase gene (37) is placed upstream from the structural gene followed again by DNA containing termination - polyadenylation signals, for example, the TRP1 (31, 32, 33) gene or the PGK (37) gene.

Because yeast 5'-flanking sequence (in conjunction with 3' yeast termination DNA) (infra) can function to promote expression of foreign genes in yeast, it seems likely that the 5'-flanking sequences of any highly-expressed yeast gene could be used for the expression of important gene products. Since under some circumstances yeast expressed up to 65 percent of its soluble protein as glycolytic enzymes (38) and since this high level appears to result from the production of high levels of the individual mRNAs (39), it should be possible to use the 5'-flanking sequences of any other glycolytic genes for such expression purposes - e.g., enolase, glyceraldehyde - 3-phosphate dehydrogenase, hexokinase, pyruvate decarboxylase, phosphofructokinase, glucose - 6-phosphate isomerase, 3-phosphoglycerate mutase, pyruvate kinase, triosephosphate isomerase, phosphoglucose isomerase, and glucokinase. Any of the 3'-flanking sequences of these genes could also be used for proper termination and mRNA polyadenylation in such an expression system - cf. Supra. Some other highly expressed genes are those for the acid phosphatases (40) and those that express high levels of production due to mutations in the 5'-flanking regions (mutants that increase expression) - usually due to the presence of a TYI transposable element (41).

All of the genes mentioned above are thought to be transcribed by yeast RNA polymerase II (41). It is possible that the promoters for RNA polymerase I and III which transcribe genes for ribosomal RNA, 5S RNA, and tRNAs (41, 42), may also be useful in such expression constructions.

Finally, many yeast promoters also contain transcriptional control so they may be turned off or on by variation in growth conditions. Some examples of such yeast promoters are the genes that produce the following proteins: Alcohol dehydrogenase II, isocytochrome-c, acid phosphatase, degradative enzymes associated with nitrogen metabolism, glyceraldehyde -3-phosphate dehydrogenase, and enzymes responsible for maltose and galactose utilization (39). Such a control region would be very useful in controlling expression of protein product - especially when their production is toxic to yeast. It should also be possible to put the control region of one 5'-flanking sequence with a 5'-flanking sequence containing a promoter from a highly expressed gene. This would result in a hybrid promoter and should be possible since the control region and the promoter appear to be physically distinct DNA sequences.

3. Cell Culture Systems/Cell Culture Vectors

Propogation of vertebrate cells in culture (tissue culture) has become a routine procedure in recent years (see 43). The COS-7 line of monkey kidney fibroblasts may be employed as the host for the production of animal interferons (44) . However, the experiments detailed here could be performed in any cell line which is capable of the replication and expression of a compatible vector, e.g., WI38, BHK, 3T3, CHO, VERO, and HeLa cell lines. Additionally, what is required of the expression vector is an origin of replication and a promoter located in front of the gene to be expressed, along with any necessary ribosome binding sites, RNA splice sites, polyadenylation site, and transcriptional terminator sequences. While these essential elements of SV40 have been exploited herein, it will be understood that the invention, although described herein in terms of a preferred embodiment, should not be construed as limited to these sequences. For example, the origin of replication of other viral (e.g., Polyoma, Adeno, VSV, BPV, and so forth) vectors could be used, as well as cellular origins of DNA replication which could function in a nonintegrated state.

B. Vector Systems

1. Direct Expression of Mature Bovine Interferon in E. coli

The procedure used to obtain direct expression of bovine interferon in E. coli as a mature interferon polypeptide (minus signal sequence) involved the combination of a plasmid containing a promoter fragment and translational start signal with a tailored fragment of animal genomic DNA that contained the coding region for the mature interferon.

2. Expression in Yeast

To express a heterologous gene such as the DNA for animal interferon in yeast, it is necessary to construct a plasmid vector containing four components. The first component is the part which allows for transformation of both E. coli and yeast and thus must contain a selectable gene from each organism, such as the gene for ampicillin resistance from E. coli and the gene TRP1 from yeast. This component also requires an origin of replication from both organisms to be maintained as a plasmid DNA in both organisms, such as the E. coli origin from pBR322 and the ars1 origin from chromosome III of yeast.

The second component of the plasmid is a 5'-flanking sequence from a highly expressed yeast gene to promote transcription of a downstream-placed structural gene, such as the 5'-flanking sequence used is that from the yeast 3-phosphoglycerate kinase (PGK) gene.

The third component of the system is a structural gene constructed in such a manner that it contains both an ATG translational start and translational stop signals. The isolation and construction of such a gene is described infra.

The fourth component is a yeast DNA sequence containing the 3'-flanking sequence of a yeast gene, which contains the proper signals for transcription termination and polyadenylation.

3. Expression in Mammalian Cell Culture

The strategy for the synthesis of immune interferon in mammalian cell culture relies on the development of a vector capable of both autonomous replication and expression of a foreign gene under the control of a heterologous transcriptional unit. The replication of this vector in tissue culture can be accomplished by providing a DNA replication origin (derived from SV40 virus), and providing helper function (T antigen) by the introduction of the vector into a cell line endogenously expressing this antigen (46, 47). The late promoter of SV40 virus preceded the structural gene of interferon and ensured the transcription of the gene.

A useful vector to obtain expression consists of pBR322 sequences which provides a selectable marker for selection in E. coli (ampicillin resistance) as well as an E. coli origin of DNA replication. These sequences are derived from the plasmid pML-1 (46) and encompasses the region spanning the EcoRI and BamHI restriction sites. The SV40 origin is derived from a 342 base pair PvuII-HindIII fragment encompassing this region (48, 49) (both ends being converted to EcoRI ends). These sequences, in addition to comprising the viral origin of DNA replication, encode the promoter for both the early and late transcriptional unit. The orientation of the SV40 origin region is such that the promoter for the late transcriptional unit is positioned proximal to the gene encoding interferon.

Brief Description of the Drawings

Figure 1 depicts a Southern hybridization of (a) human, (b) bovine and (c) porcine genomic DNAs digested with EcoRI and hybridized at different formamide concentrations with a 32P-labelled 570 base-pair EcoRI fragment containing the coding region of the human leukocyte interferon A/D hybrid. The hybridization at 20 percent formamide gives the clearest pattern of the mutigene bovine and porcine leukocyte interferon gene families.

Figure 2 depicts a Southern hybridization of four different bovine genomic DNA phage recombinants digested with EcoRI, BamHI or HindIII and hybridized with a $^{32}$P-labelled human leukocyte gene probe. Clone 83 yields two hybridizing fragments with each restriction enzyme.

Figure 3A shows a portion of the nucleotide sequence from the plasmid subclone p83BamHI1.9kb as well as the deduced amino acid sequence for the bovine leukocyte interferon coded therein. The signal peptide is represented by amino acid residues S1 through S23.

Figure 3B shows the nucleotide sequence and deduced amino acid sequence for a second bovine leukocyte interferon ($\alpha$2) from the plasmid subclone p67EcoRI 3.2 kb.

Figure 3C shows the complete mature nucleotide sequence and deduced amino acid sequence for a third bovine leukocyte interferon ($\alpha$3) from the plasmid subclone p35EcoRI-BamHI 3.5 kb.

Figure 3D shows the nucleotide sequence and deduced amino acid sequence for a fourth bovine leukocyte interferon hereof from the plasmid subclone p83EcoRI-BamHI 2.9 kb. The signal is represented by amino acid residues S1 to S23. The mature protein comprises 172 amino acid residues. It is noted that the last stretch of six amino acid residues is attributed to a nucleotide base change at position 511 which allows six additional translatable codons before the next in phase stop signal.

Figure 4 compares the amino acid sequences of BoIFN-$\alpha$1, $\alpha$2, $\alpha$3 and $\alpha$4 with the sequences for 11 known human leukocyte interferons. Also given are the amino acids conserved among all human leukocyte interferons, all bovine leukocyte interferons, and with respect to bovine $\alpha$1 and $\alpha$4, positions where homology with the majority of human leukocyte interferons occur.

Figure 5 is a schematic diagram of the construction of the bovine leukocyte interferon expression plasmid pBoIFN-$\alpha$1trp55. The starting materials are the trp expression vector pdeltaRIsrc and the BamHI fragment from the plasmid subclone p83BamHI1.9kb.

Figure 6 depicts a Southern hybridization of bovine DNA digested with either EcoRI, HindIII, BamHI, BgIII and PvuII with a radioactive probe prepared from BoIFN-$\alpha$1 or BoIFN-$\alpha$4 gene fragments. Each IFN gene preferentially hybridizes with a distinct subfamily of BoIFN-$\alpha$ genes.

Figure 7 shows a restriction map of the genomic bovine DNA inserts from three phage recombinants which hybridize the human fibroblast human interferon gene probe. The location and orientation of each BoIFN-$\beta$ is indicated by the black rectangle. Restriction sites marked by an asterisk represent partial restriction mapping information.

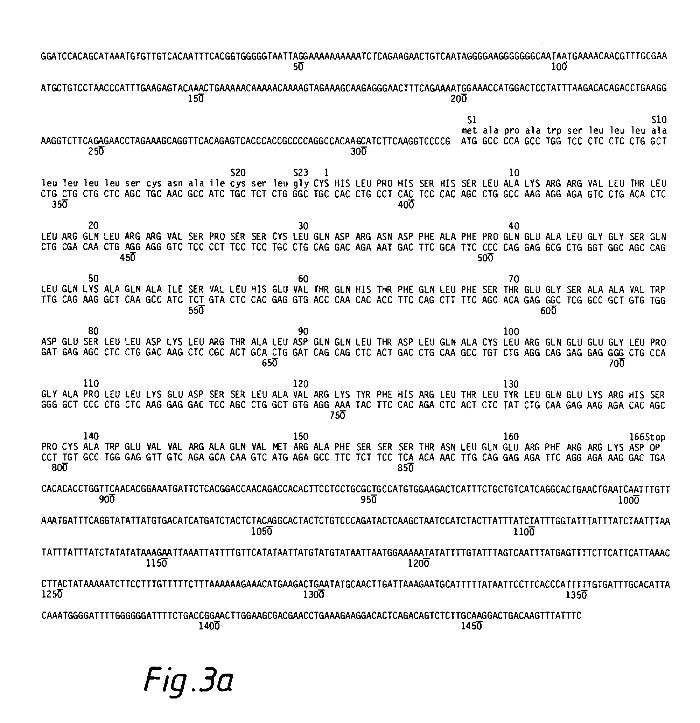

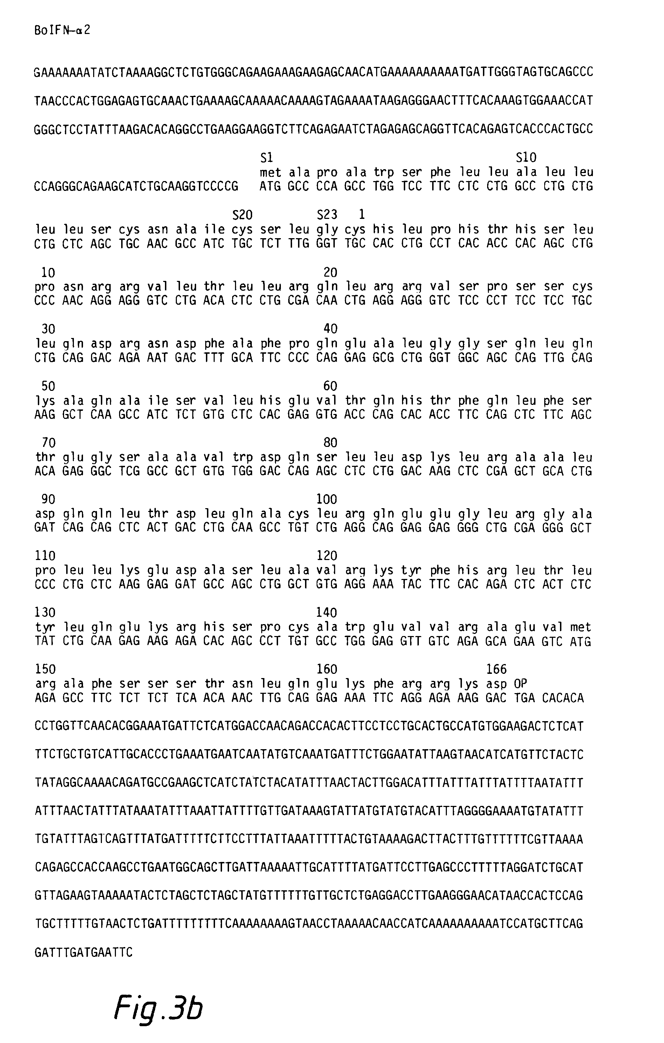

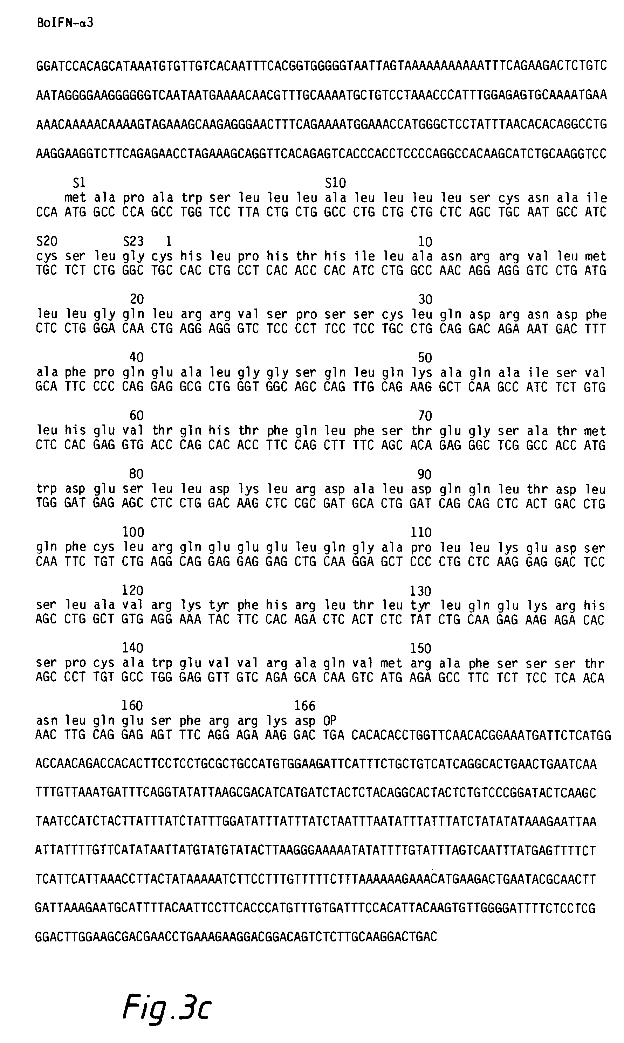

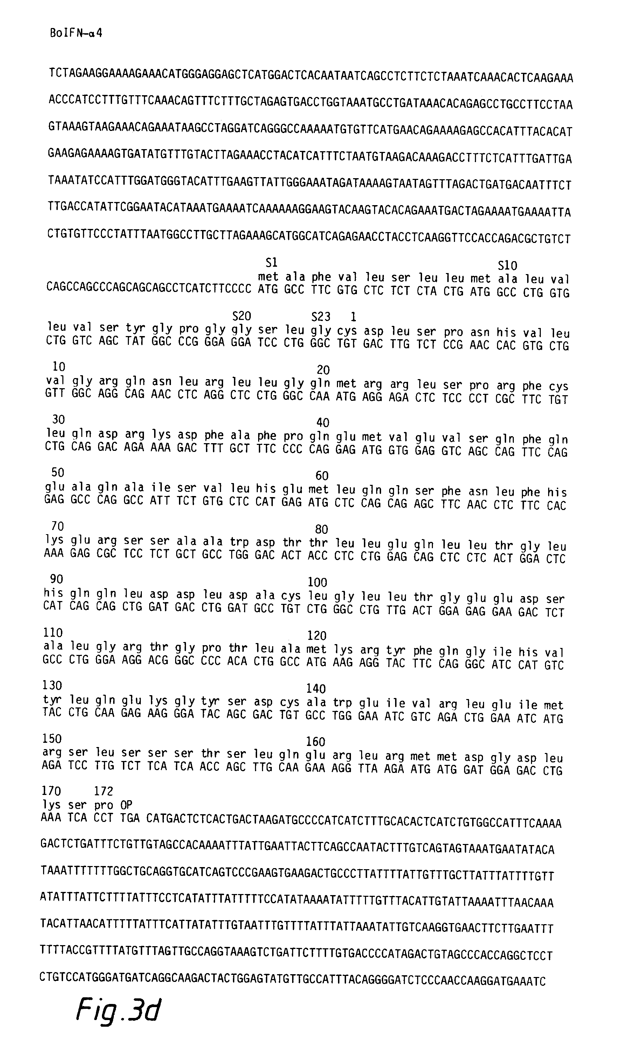

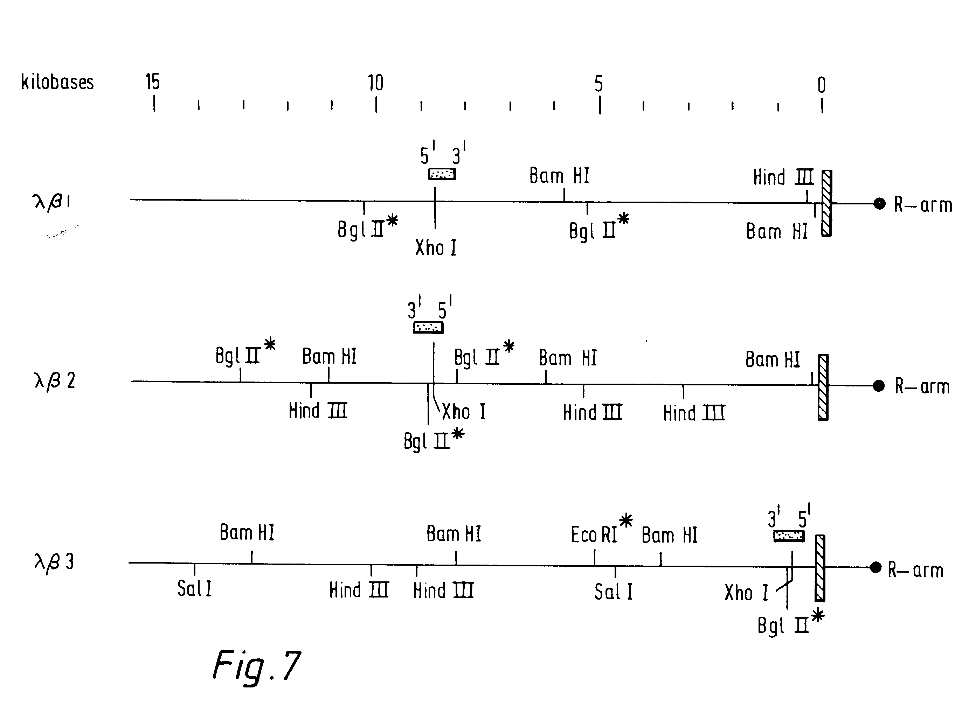

Figure 3 shows a finer resolution restriction map for the three genes referenced in Figure 7. Hatching represents the signal sequence; shading, the mature sequence.

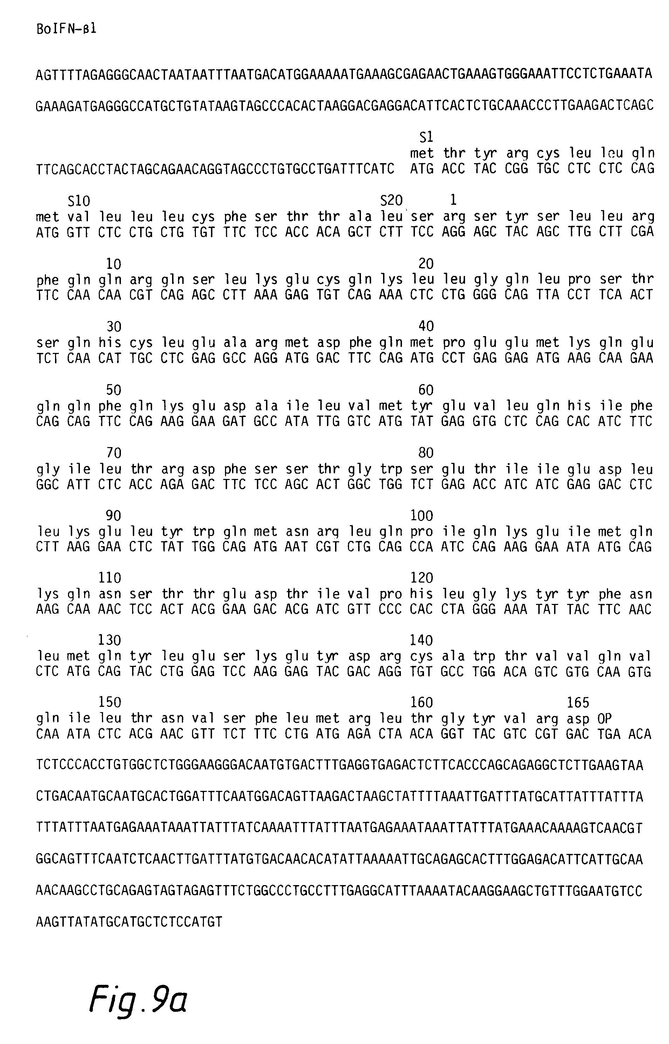

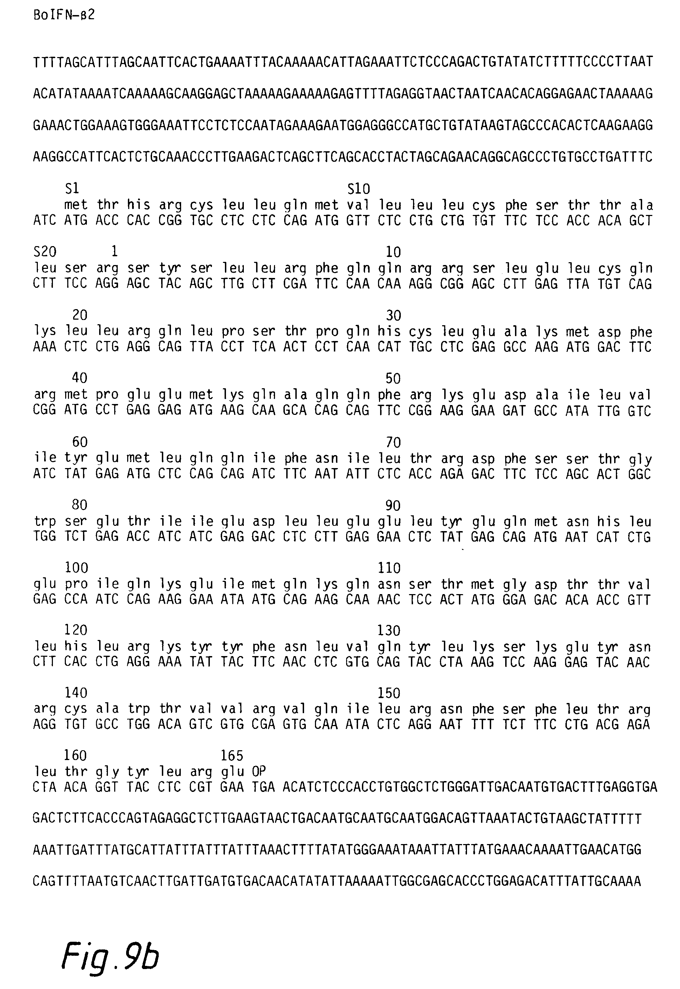

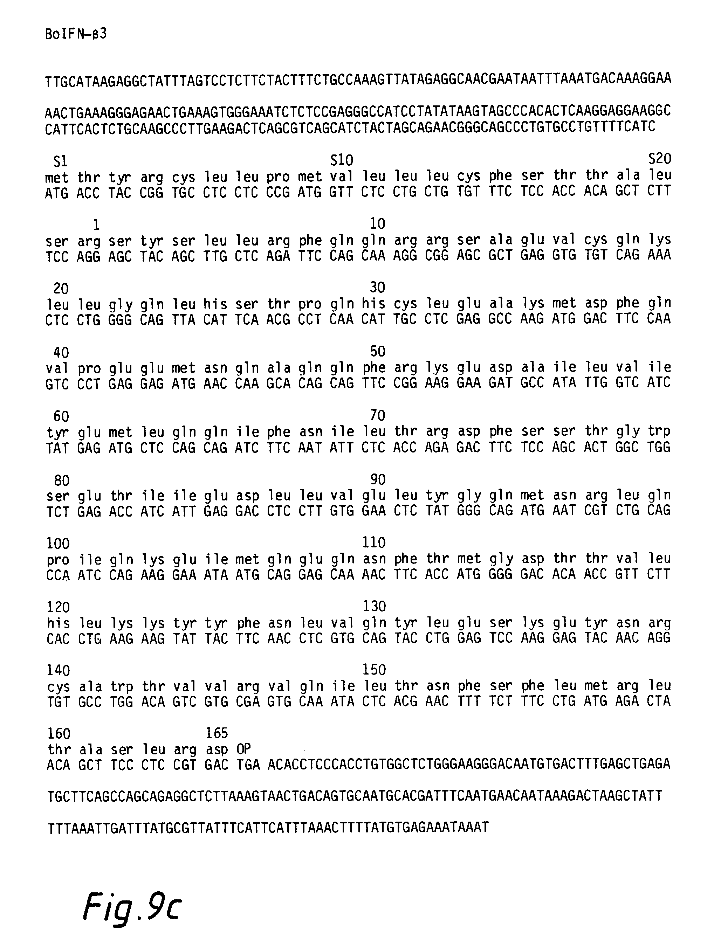

Figures 9a, 9b and 9c depict the nucleotide and deduced amino acid sequences for the BoIFN-$\beta$1, 2 and 3 genes.

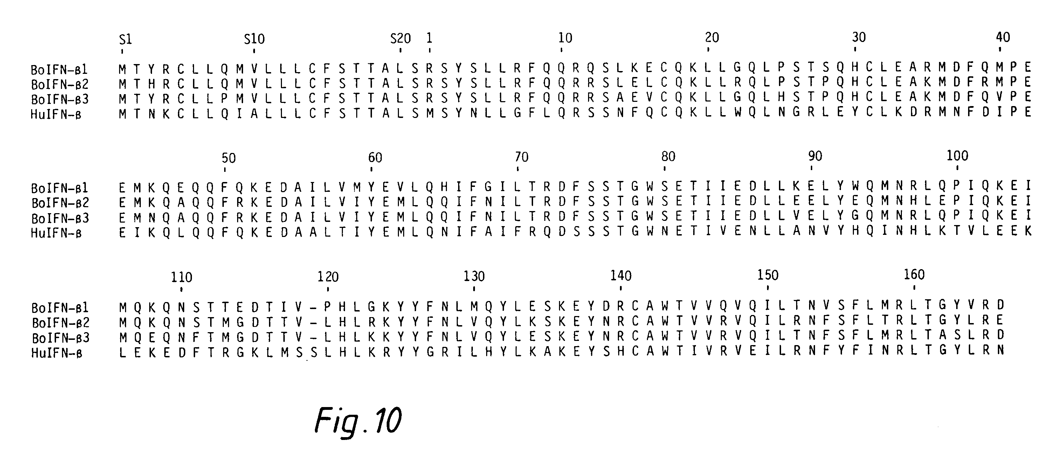

Figure 10 compares the amino acid sequences for the three BoIFN-$\beta$s with HuIFN-$\beta$.

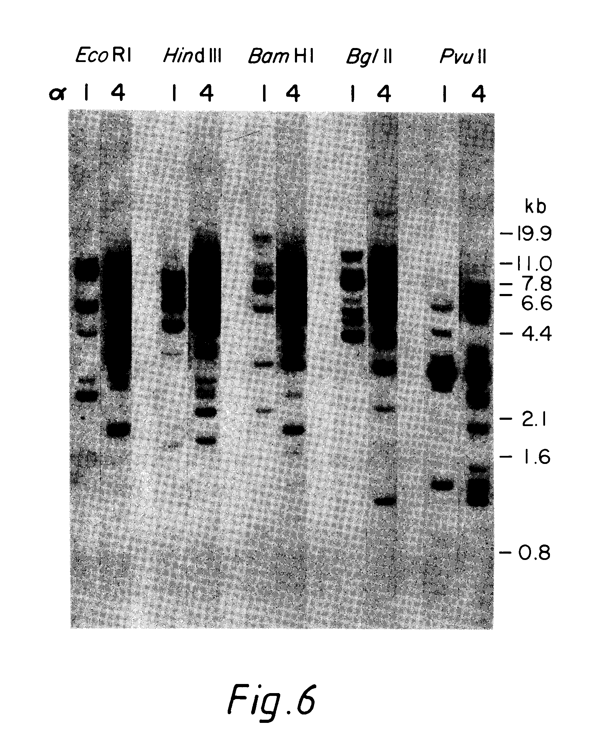

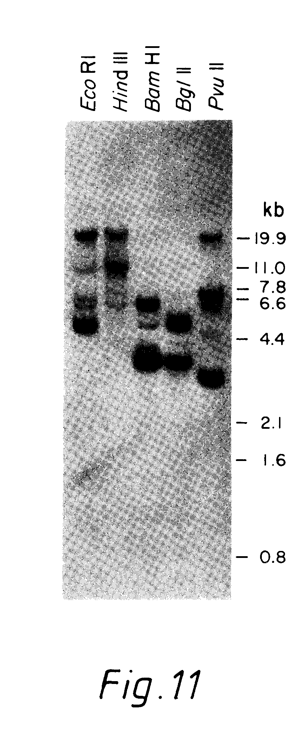

Figure 11 is a Southern blot of Figure 6 rehybridized with a BoIFN-$\beta$1 gene probe under conditions in which only a single hybridizing fragment would, in general, become apparent when performing an analogous experiment with human genomic DNA and the HuIFN-$\beta$ gene (9).

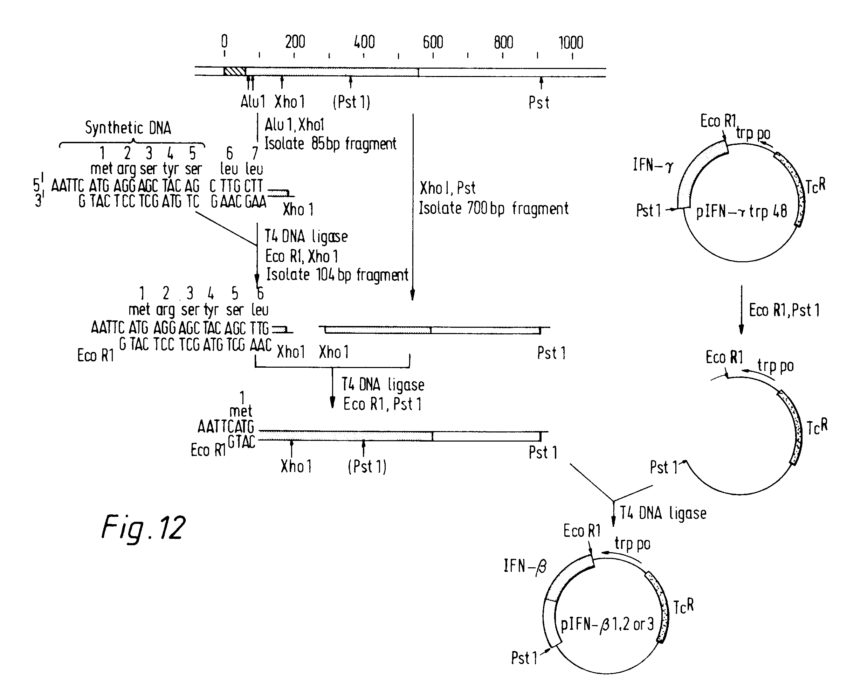

Figure 12 schematically depicts the strategy used to express all three BoIFN-$\beta$s under control of the trp operon of E. coli.

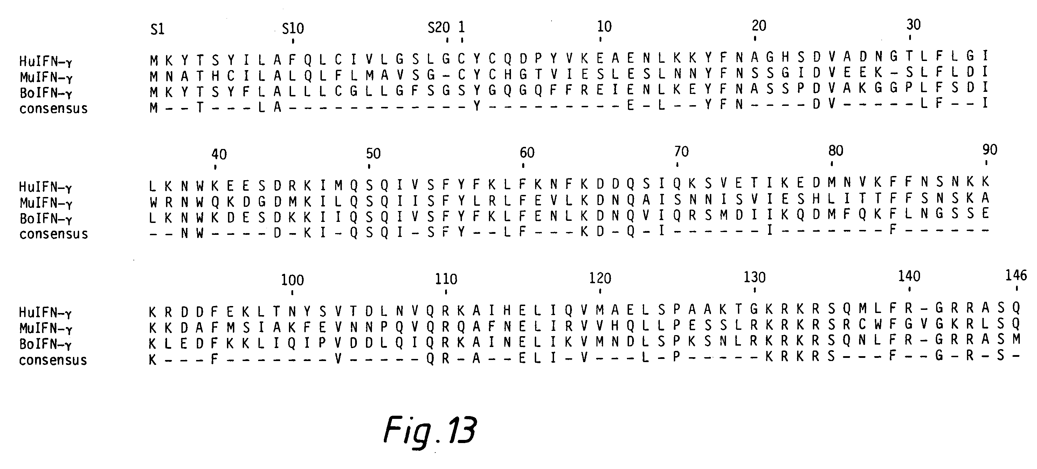

Figure 13 gives the comparison of the deduced amino acid sequences of BoIFN-$\gamma$, HuIFN-$\gamma$ and murine IFN-$\gamma$.

Detailed Description

The following detailed description is illustrative of the invention for the preparation, via recombinant DNA technology, of the various animal interferons embraced, and sets forth generally applicable methodology for the preparation of particular, bovine leukocyte interferons. The method is described with respect to a bacterial system.

A. Isolation of Bovine DNA

For the purpose of constructing an animal gene library, high molecular weight DNA was isolated from animal tissue by a modification of the Blin and Stafford procedure (50), randomly fragmented with respect to gene locus, and size fractionated to obtain 15-20 kilobase fragments for cloning into a lambda phage vector (51).

Frozen tissue, for example bovine pancreas, was ground to a fine powder in liquid nitrogen and solubilized in 0.25 M EDTA, 1 percent Sarkosyl, 0.1 mg/ml Proteinase K (25 ml/gram tissue) at 50 °C for 3 hours. The viscous solution obtained was deproteinized by three phenol and one chloroform extractions, dialysed against 50 mM Tris-HCl (pH8), 10 mM EDTA, 10 mM NaCl and digested with heat-treated pancreatic ribonuclease (0.1 mg/ml) for 2 hours at 37 °C. After phenol and ether extraction, the DNA was precipitated with two volumes of ethanol, washed in 95 percent ethanol, lyophilized and redissolved in TE buffer (10 mM Tris-HCl (pH 7.5), 1 mM EDTA) overnight at 4 °C at a final concentration of 1-2 mg/ml. The final DNA preparation was greater than 100 kilobases in length as determined by electrophoresis on a 0.5 percent neutral agarose gel.

B. Partial Endonuclease Digestion and Size Fractionation of Bovine DNA

Aliquots (0.1 mg) of bovine DNA were digested with 1.25, 2.5, 5 and 10 units of Sau3A at 37°C for 60 minutes in a reaction (1 ml) containing 10 mM Tris-HCl (pH 7.5), 10 mM MgC 12, 2 mM dithiothreitol. Incubations were stopped by adding EDTA to 25 mM, phenol and ether extracted, made 0.3 M in sodium acetate (pH 5.2) and precipitated with 3 volumes of ethanol. The DNA was redissolved in TE buffer at 68°C and sedimented through a 10-40 percent linear sucrose gradient (51) in a Beckman SW 27 rotor at 27,000 rpm for 22 hours at 20°C. Fractions (0.5 ml) were analyzed on a 0.5 percent gel using Eco R1-digested Charon 4A (51a) DNA as a molecular weight standard. Those fractions containing 15-20 kilobase DNA fragments were combined, precipitated with ethanol and redissolved in TE buffer.

C. Construction of the Bovine Genomic DNA Library

The 15-20 kb bovine DNA nonlimit digest was cloned into a lambda Charon 30 A vector (52) having G-A-T-C sticky ends generated by removal of the two internal Bam HI fragments of the phage. Charon 30 A was grown in E. coli strain DP 50 SupF (ATTC No. 39061, deposited March 5, 1982) in NZYDT broth, concentrated by polyethylene glycol precipitation and purified by CsCl density gradient centrifugation (53). Phage DNA was prepared by extracting the purified phage twice with phenol, once with phenol and ether, and concentrating the DNA by ethanol precipitation.

For preparation of the end fragments of Charon 30A, 50 micrograms of phage DNA was annealed for 2 hours at 42°C in 0.25 ml of 50 mM Tris-HCl (pH 8), 10 mM MgC12 and 0.15 M NaCl, digested to completion with Bam HI, phenol and ether extracted, and sedimented through a 10 to 40 percent sucrose gradient as described above. Fractions containing the 32 kb annealed arms of the phage were combined and ethanol precipitated.

The purified Charon 30 A arms (6 micrograms) were reannealed at 42 °C for 2 hours, combined with 0.3 micrograms of 15-20 kb bovine DNA an 400 units of phage T4 polynucleotide ligase and incubated overnight at 12°C in a 0.075 ml reaction containing 50 mM Tris-HCl (pH 7.5), 10 mM MgC12, 20 mM dithiothreitol and 50 micrograms/ml bovine serum albumin. The ligated DNA mixture was then packaged into mature lambda phage particles using an in vitro lambda packaging system (54).

The components of this system-sonic extract (SE), freeze-thaw lysate (FTL), protein A, and buffers A and M1-were prepared as described (54). Three microliter aliquots of the ligated DNA mixture were incubated with 15 microliters of Buffer A, 2 microliters of Buffer M1, 10 microliters of SE and 1 microliter of protein A for 45 minutes at 27°C. The FTL was thawed on ice for 45 minutes, combined with 0.1 volumes of Buffer M1, centrifuged at 35,000 rpm at 4°C for 25 minutes, and 0.075 ml aliquots of the supernatant were added to the above reaction. After an additional 2 hours of incubation at 27°C, a small aliquot of the packaging reaction was titered on strain DP 50 SupF, supra. This procedure yielded a total of approximately $1.1 \times 10^6$ independent bovine DNA recombinants. The remainder of the packaging mixture was amplified by a plate-lysate method (52) by plating out the recombinants on DP 50 SupF at a density of 10,000 plaque-forming units per 15 cm NZYDT agar plate.

D. Screening of the Phage Library for Bovine Interferon Genes

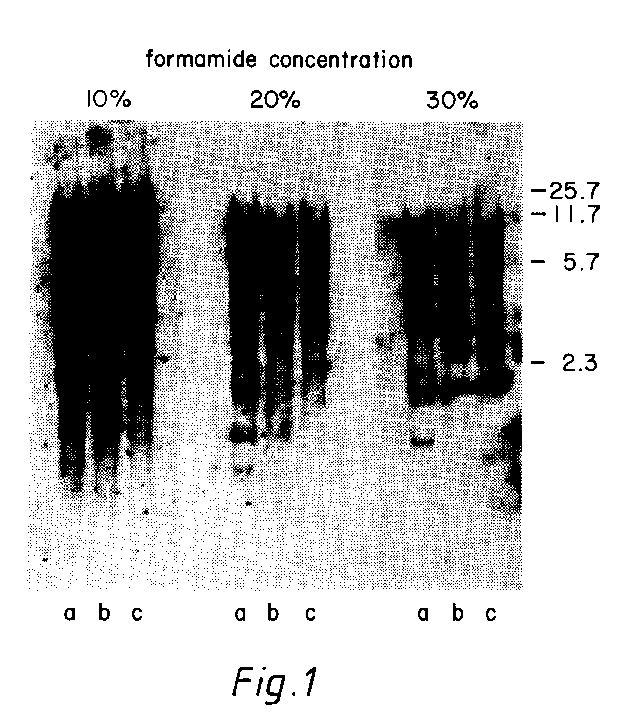

The strategy used to identify phage recombinants carrying bovine interferon genes consisted in detecting nucleotide homology with radioactive probes prepared from cloned human leukocyte (8,9), fibroblast (12) and immune (55) interferon genes. Hybridization conditions were established with Southern blots (56) of genomic animal DNA. Five micrograms each of high molecular weight DNA (prepared as described above) from human placenta, bovine pancreas and pig submaxillary gland were digested to completion with Eco RI, electrophoresed on a 0.5 percent agarose gel and transferred to nitrocellulose paper (56). A 32P-labelled DNA probe was prepared from a 570 base-pair Eco R1 fragment containing the protein coding region of the mature human leukocyte interferon A/D hybrid at the Bgl II restriction site (57) by standard procedures (58) . Each nitrocellulose filter was prehybridized at 42°C overnight in 5xSSC (56), 50 mM sodium phosphate (pH 6.5), 0.1 mg/ml sonicated salmon sperm DNA, 5x Denhardt's solution (59), 0.1 percent sodium dodecyl sulfate, 0.1 percent sodium pyrophosphate that contained either 10 percent, 20 percent, or 30 percent formamide, and then hybridized with $100 \times 10^6$ counts per minute of the labelled probe in the same solution containing 10 percent sodium dextran sulfate (60). After an overnight incubation at 42°C, the filters were washed 4 times in 2xSSC, 0.1 percent SDS at room temperature, once in 2xSSC and then exposed to Kodak XR-5 x-ray film with Dupont Cronex intensifying screens overnight. As seen in Figure 1, a number of hybridizing bands are most readily detected in the bovine and porcine DNA digests when 20 percent formamide is present in the hybridization. This result provides evidence for a multigene family of leukocyte interferon genes in cow and pig analogous to that previously demonstrated in humans (12,61). The same hybridization conditions were therefore employed to screen for interferon genes in the bovine DNA library.

500,000 recombinant phage were plated out on DP 50 SupF at a density of 10,000 pfu/15 cm plate, and duplicate nitrocellulose filter replicas were prepared for each plate by the method of Benton and Davis (62). The filters were hybridized with the human LeIF gene probe as described above. Ninety-six duplicate hybridizing plaques were obtained which gave strong signals upon repeated screening.

The bovine library was further screened for fibroblast and immune interferon genes. Probes were made from a 502 base-pair Xba I-Bgl III fragment containing the entire mature human fibroblast interferon gene (12), and a 318 base-pair Alu I fragment (containing amino acids 12-116) and 190 base-pair Mbo II fragment (containing amino acids 99-162) from the mature coding region of the human immune interferon gene (55). Hybridization of $1.2 \times 10^6$ recombinant phage yielded a total of 26 bovine fibroblast and 10 bovine immune interferon clones.

### E. Characterization of the Recombinant Phage

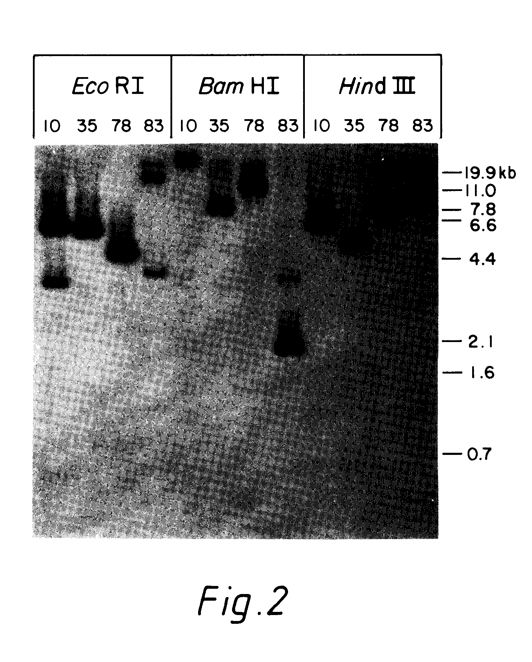

Phage DNA was prepared (as described above) from 12 recombinants which hybridized with the human leukocyte interferon probe. Each DNA was digested singly and in combination with Eco R1, Bam HI and Hind III, electrophoresed on a 0.5 percent agarose gel and the location of the hybridizing sequence mapped by the Southern method (56). A comparison of singly digested DNA from clones 10, 35, 78 and 83 is shown in Figure 2. For each phage the sizes of restriction fragments observed as well as the corresponding hybridization pattern is distinct and nonoverlapping, suggesting that each of these four phage carry a different bovine interferon gene. In addition, digestion of clone 83 with each of the three enzymes yields in each case two discrete hybridizing bands, indicating that this recombinant may carry two closely linked interferon genes.

### F. Subcloning of the Bovine Leukocyte Interferon Genes

Restriction fragments from three of the recombinant phage which hybridized with the human leukocyte gene probe were subcloned into the multiple restriction enzyme cloning site of the pBR322 derivative, pUC9. The plasmid pUC9 was derived from pBR322 by first removing the 2,067 base-pair EcoRI-PvuII fragment containing the tetracycline resistance gene, then inserting a 425 base-pair HaeII fragment from the phage M13 derivative mP9 (62a) into the HaeII site of the resulting plasmid at position 2352 (relative to the pBR322 notation). The HaeII fragment from mp9 contains the N-terminal coding region of the E. coli lacZ gene in which a multi-restriction enzyme cloning site of the sequence, CCA AGC TTG GCT GCA GGT CGA CGG ATC CCC GGG, has been inserted between the 4th and 5th amino acid residues of $\beta$-galactosidase. Insertion of a foreign DNA fragment into these cloning sites disrupts the continuity between the lac promotor and lacZ gene, thus altering the phenotype of a JM83 transformed with the plasmid from lac$^+$ to lac$^-$.

The fragments referred to above were: (a) a 1.9 kb Bam HI fragment and 3.7 kb EcoRI fragment from clone 83 (which corresponds to nonoverlapping segments of the same recombinant), (b) a 3.5 kb BamHI-EcoRI fragment from clone 35, and (c) a 3.2 kb EcoRI fragment from clone 67. In each case, 0.1 micrograms of the appropriately digested vector was ligated with a tenfold molar excess of the purified fragment, transformed into E. coli strain JM83 (ATCC No. 39062, deposited March 5, 1982), plated out onto M9 (63) plates containing 0.04 mg/ml 5-bromo-4-chloro-3-indolyl-$\beta$-D-galactoside and 0.2 mg/ml ampicillin. White colonies, which presumably carry a DNA insert at a restriction site interrupting the coding region of the lacZ gene on pUC9, were picked into 5 ml of LB broth plus 0.02 mg/ml ampicillin, grown for several hours at 37°C, and screened for the inserted fragment by a plasmid DNA minipreparation procedure (64).

### G. DNA Sequence of a Bovine Leukocyte Interferon Gene on Clone 83

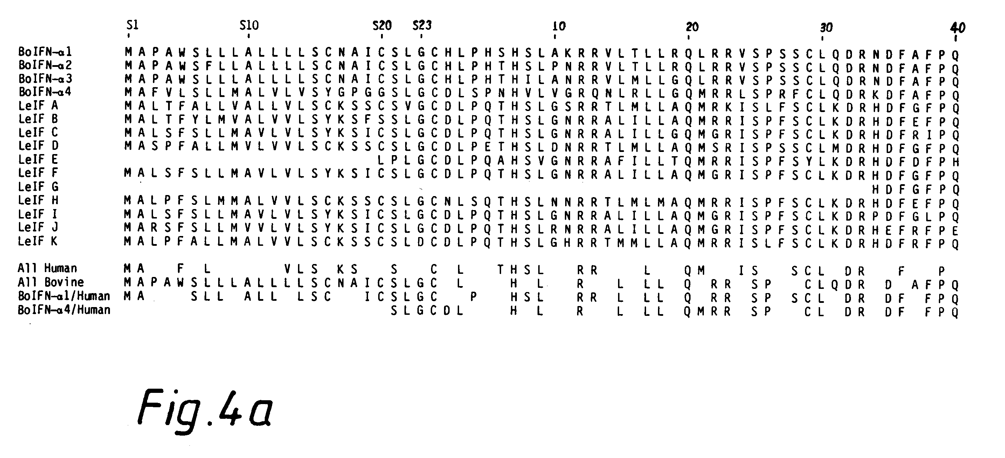

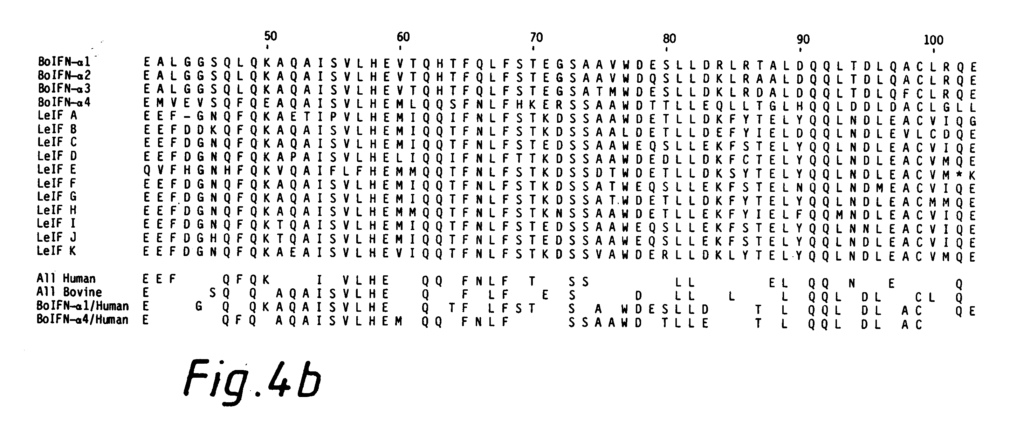

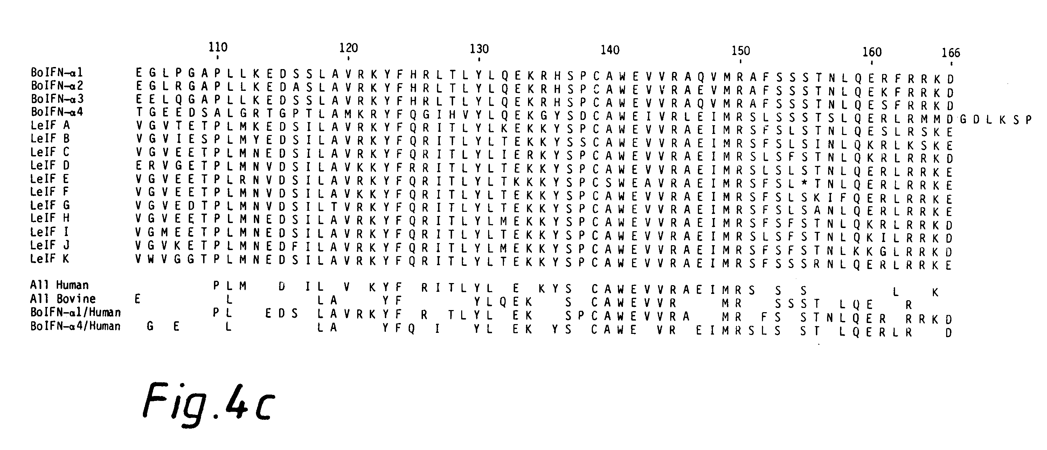

The DNA sequence extending from th Bam HI site of p83BamHI1.9kb (the 1.9 kb fragment subclone of clone 83) was determined by the Maxam-Gilbert chemical procedure (65), and is presented in Figure 3. The longest open reading frame encodes a polypeptide of 189 amino acids with significant homology to the human leukocyte interferons (Figure 4). By analogy with the human proteins, the bovine leukocyte interferon consists of a hydrophobic 23 amino acid signal peptide which precedes a 166 amino acid mature protein by an identical sequence, ser-leu-gly-cys. Four cysteine residues at positions 1, 29, 99 and 139 are exactly conserved between species. A pairwise homology comparison between the bovine and human interferons is shown in Table 1. As may be expected, the bovine protein is significantly less homologous (approximately 60 percent) to each of the human proteins than the latter are to one another (greater than 80 percent).

The DNA sequence and deduced amino acid sequence for three additional bovine leukocyte interferon genes occurring on the plasmid subclones p67EcoRI 3.2 kb, p35EcoRI-BamHI 3.5 kb and p83EcoRI 3.7 kb are shown in Figures 3B, 3C and 3D, respectively.

As summarized in Table 1, whereas the BoIFN-α2 and 3 genes encode peptides with only minor apparent differences to BoIFN-α1, the BoIFN-α4 protein is as distinct from the other bovine peptides as are any two bovine and human leukocyte interferons.

To ascertain whether the α4 gene derives from as broad a class of cellular proteins as the other BoIFN-αs, genomic bovine DNA was digested with several restriction endonucleases and hybridized with radioactive DNA fragments representing the protein coding regions of the α1 (612 bp AvaII fragment, Figure 6) and α4 (EcoRI-XmnI fragment of pBoIFN-α4trp15) genes, under conditions of high stringency (50 percent formamide) that do not allow cross hybridization of the two genes. As seen in Figure 6, each gene preferentially hybridizes to a distinct set of bovine DNA fragments . These results together clearly demonstrate the existence of two different families of bovine leukocyte IFN peptides, of which the α1 and α4 proteins may be thought of as representative members.

Table 1

Pairwise comparisons of differences

in coding sequences of bovine and human IFN-αs

| | α1 | α2 | α3 | α4 | αA | αB | αC | αD | αF | αH | αI | αJ | αK |
|---|---|---|---|---|---|---|---|---|---|---|---|---|---|
| BoIFN-α1 | | 94 | 92 | 54 | 61 | 62 | 63 | 64 | 61 | 64 | 63 | 61 | 65 |
| BoIFN-α2 | 96 | | 91 | 53 | 61 | 61 | 64 | 63 | 62 | 63 | 63 | 64 | 64 |
| BoIFN-α3 | 100 | 96 | | 45 | | | | | | | | | |
| BoIFN-α4 | 52 | 48 | 52 | | 54 | 54 | 58 | 55 | 56 | 58 | 56 | 54 | 54 |
| HuIFN-αA | 56 | 52 | | 39 | | 81 | 81 | 83 | 82 | 83 | 81 | 80 | 86 |
| HuIFN-αB | 43 | 39 | | 48 | 70 | | 81 | 77 | 81 | 83 | 80 | 79 | 81 |
| HuIFN-αC | 61 | 57 | | 52 | 70 | 65 | | 81 | 89 | 86 | 94 | 92 | 83 |
| HuIFN-αD | 52 | 48 | | 48 | 74 | 61 | 65 | | 83 | 81 | 80 | 78 | 84 |
| HuIFN-αF | 61 | 57 | | 52 | 70 | 65 | 100 | 65 | | 83 | 89 | 86 | 83 |
| HuIFN-αH | 56 | 52 | | 52 | 74 | 74 | 74 | 83 | 74 | | 84 | 84 | 84 |
| HuIFN-αI | 61 | 57 | | 52 | 70 | 65 | 100 | 65 | 100 | 74 | | 91 | 81 |
| HuIFN-αJ | 56 | 52 | | 48 | 61 | 57 | 91 | 70 | 91 | 65 | 91 | | 80 |
| HuIFN-αK | 52 | 48 | | 48 | 83 | 70 | 74 | 91 | 74 | 91 | 74 | 65 | |

Numbers represent percentage homology

Lower-left half represents 23 acid presequence

Upper-right half represents 166 acid mature protein

A,B,C, etc. are human leukocyte interferons A,B,C, etc.

H. Direct Expression of Mature BoIFN-α1 in E. coli

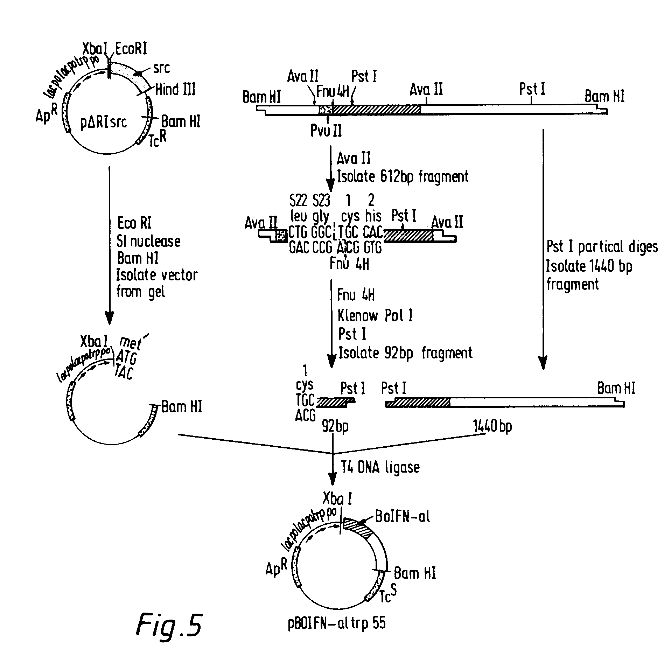

The construction of the direct expression plasmid is summarized in Figure 5. The plasmid subclone p83BamHI1.9kb was digested to completion with Ava II, and the 612 base-pair fragment containing the bovine leukocyte interferon gene isolated by electrophoresis on a 6 percent polyacrylamide gel and electroeluted. Approximately 1.5 micrograms of this fragment was digested with Fnu4H, phenol and ether extracted, and ethanol precipitated. The resulting Fnu4H sticky ends were extended to blunt ends with 6 units of DNA polymerase I (Klenow fragment) at 12°C for 30 minutes in 20 microliters containing 20 mM Tris-HCl (pH 7.5), 10 mM $MgCl_2$, 4 mM dithiothreitol and 0.1 mM each dATP, dGTP, dCTP and dTTP. After extraction with phenol and ether, the DNA was digested with Pst I and electrophoresed on a 6 percent gel. The resulting 92 base-pair blunt end-Pst I fragment which extends from the first nucleotide of the coding region for the mature bovine leukocyte interferon was electroeluted from the gel.

The remainder of the mature coding region was isolated as follows. Three micrograms of the Bam HI insert of p83BamHI1.9kb was partially digested with 14 units of Pst I for 10 minutes at 37°C in a 45 microliter reaction containing 10 mM Tris-HCl (pH 7.5), 10 mM $MgCl_2$, 2 mM dithiotreitol, and extracted with phenol and ether. The desired 1440 base-pair partial Pst I-Bam HI fragment extending from nucleotide 93 of the mature coding region was isolated from a 6 percent polyacrylamide gel.

The plasmid pdeltaRIsrc is a derivative of the plasmid pSRCex16 (66) in which the Eco RI sites proximal to the trp promoter and distal to the src gene have been removed by repair with DNA polymerase I (67), and the self-complementary oligodeoxynucleotide AATTATGAATTCAT (synthesized by the phosphotriester method (68) was inserted into the remaining Eco RI site immediately adjacent to the Xba I site. 20 micrograms of pdeltaRIsrc was digested to completion with Eco RI, phenol and ether extracted, and ethanol precipitated. The plasmid was then digested with 100 units of nuclease S1 at 16°C for 30 minutes in 25 mM sodium acetate (pH 4.6), 1 mM $ZnCl_2$ and 0.3 M NaCl to create a blunt end with the sequence ATG. After phenol and ether extraction and ethanol precipitation, the DNA was digested with Bam HI, electrophoresed on a 6 percent polyacrylamide gel, and the large (4300 bp) vector fragment recovered by electroelution.

The expression plasmid was assembled by ligating together 0.2 micrograms of vector, 0.02 micrograms of the 92 bp blunt-Pst I fragment and 0.25 micrograms of the 1400 bp partial Pst I - Bam HI fragment with 400 units of T4 DNA ligase overnight at 12°C, and used to transform E. coli strain 294 (ATCC No. 31446) to ampicillin resistance. Plasmid DNA was prepared from 96 of the colonies and digested wit Xba I and Pst I. Nineteen of these plasmids contained the desired 103 base-pair XbaI-PstI and 1050 base-pair Pst I fragments. DNA sequence analysis verified that several of these plasmids had an ATG initiation codon correctly placed at the start of the bovine interferon coding region. One of these plasmids, pBoIFN-α1trp55 was chosen for further study.

I. Direct Expression of a Second Class of Mature Bovine Leukocyte Interferon (α-4) in E. coli

An ATG initiation codon was placed in front of the mature coding region by the enzymatic extension of a synthetic DNA primer, CATGTGTGACTTGTCT. The heptadecamer was phosphorylated with T4 polynucleotide kinase and γ-32P ATP as previously described (12). 250 pmoles of the primer was combined with approximately 1 microgram of a 319 bp HincII fragment containing amino acid residues S20 to 102 in 30 microliters of $H_2O$, boiled for 5 min, and extended with 25 units of E. coli DNA polymerase I Klenow fragment at 37°C for 3 hours. The product of this reaction was digested with HgiAI and the resulting 181 bp blunt-HgiAI fragment was isolated from a 6 percent polyacrylamide gel.

The entire gene for the mature peptide was assembled behind the trp promoter by enzymatically ligating the above fragment with a 508 bp HgiA-PstI fragment containing the carboxy-terminal portion of the peptide and the HuIFN-γ expression plasmid, pIFN-γtrp48-13 (55), which had been digested with EcoRI, extended to flush ends with Klenow DNA polymerase, digested with PstI and finally isolated on a 6 percent polyacrylamide gel. Upon transformation of resulting mixture into E. coli 294, several clones were identified which had restored the EcoRI recognition site between the trp promoter-ribosome binding site region of the parent expression vector and the complete coding region of the mature bovine IFN (pBoIFN-α4trp15).

J. Subcloning of the Bovine Fibroblast Interferon Genes

Six of the phage recombinants which hybridized with the human IFN-β DNA probe were purified and their DNA isolated as described above for further analysis. Restriction mapping combined with Southern hybridization analysis indicated that the six isolates comprised three distinct regions of the bovine genome, thus implying a multigene BoIFN-$\beta$ family. These results are summarized by the restriction maps shown in Figure 7. To obtain a more detailed restriction map and nucleotide sequence for each distinct class of recombinant, hybridizing fragments were subcloned into plasmid vectors. Specifically, the 5kb BglII fragment of phage $\lambda\beta$1 and the 5 kb BamHI fragment of phage $\lambda\beta$2 were individually cloned into pBR322 at the BamHI site, the overlapping 4.5 kb EcoRI-XhoI and 1.4 kb PstI-HpaI fragment of phage $\lambda\beta$3 were inserted into pUC9 (deleted from the EcoRI-SalI sites) and pLeIF87 (10) (deleted from HpaI-PstI), respectively.

K. DNA Sequences of Three Distinct Bovine Fibroblast IFN Genes

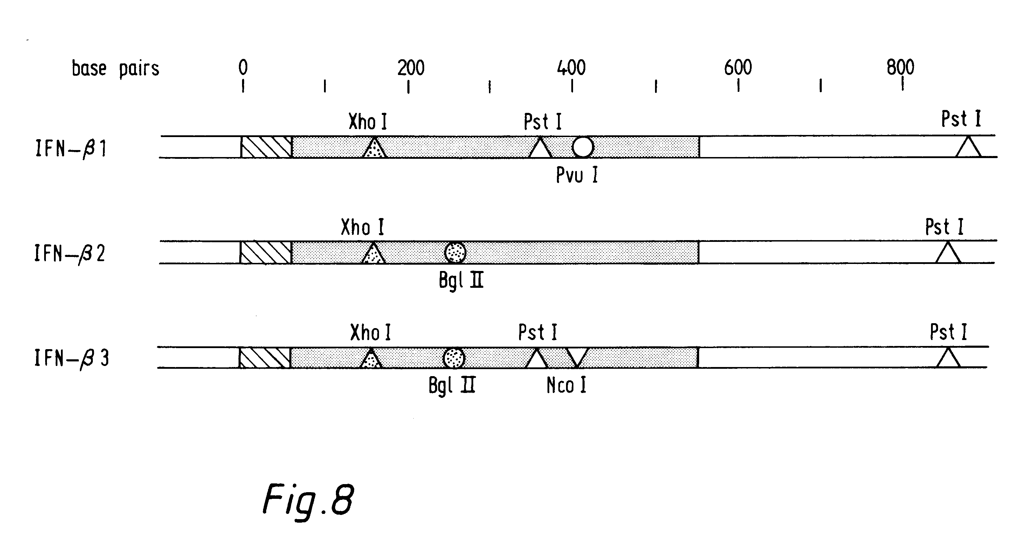

Figure 8 shows restriction maps for each of the three types of bovine IFN-$\beta$ genes that were subcloned. These are easily distinguished by the presence of cleavage sites unique to each. The peptide coding regions as well as sequences immediately upstream and downstream for each gene was was determined by the Maxam-Gilbert chemical procedure and are shown in Figures 9a, 9b and 9c. Nucleotide homology with the sequence determined for the human fibroblast interferon gene (12) predicts the correct reading frame and entire amino acid sequence for each bovine gene product, which includes a hydrophobic signal peptide of 21 amino acids followed by a mature protein of 185 residues. The bovine proteins are quite distinct from one another (Table 2, Figure 10), but show an even greater difference (approximately 60 percent) with the human peptide.

The multigene nature of bovine fibroblast interferon was further demonstrated by rehybridizing the Southern blot shown in Figure 11, with a radioactive probe prepared from a 415 bp EcoRI-PvuI fragment derived from pBoIFN-$\beta$1trp (described below). As seen in Figure 11, this experiment provides evidence for the existence of additional, homologous IFN-$\beta$genes. The lesser hybridizing bands may in fact represent more distantly related genes, that would in turn encode more distinct $\beta$-IFNs.

Table 2

| Pairwise Comparisons of Homology in Coding Sequences of Bovine IFN-$\beta$s and the Human IFN-$\beta$. | | | | |
|---|---|---|---|---|
|  | $\beta$1 | $\beta$2 | $\beta$3 | Hu$\beta$ |
| $\beta$1 |  | 138 (83) | 138 (83) | 84 (51) |
| $\beta$2 | 20 (95) |  | 146 (88) | 92 (55) |
| $\beta$3 | 20 (95) | 19 (90) |  | 87 (52) |
| Hu$\beta$ | 16 (76) | 17 (81) | 16 (76) |  |
| The number of identical amino acids in each pair of coding sequences are shown. The 21 amino acid signal peptide are compared in the lower left part and the 166 amino acid mature IFN-$\beta$s are compared in the upper right part of the table. The total number of identical amino acids in each pair is listed first, followed by the percentage homology. | | | | |

L. Direct Expression of Three Bovine IFN-$\beta$s in E. coli

As the three bovine IFN-$\beta$ genes share many common DNA sequences and restriction sites (see Figure 8), a general scheme is feasible for the expression of all three genes. Since the DNA sequence coding for the first five amino acids, which contains two AluI sites, was identical in each case, two complementary synthetic oligonucleotides were designed which incorporate an ATG translational initiation codon, restore the codons for the first 4 amino acids of mature bovine IFN-$\beta$, and create an EcoRI sticky for insertion after a trp promoter sequence. Construction of the expression plasmids is schematized in Figure 12. Ligation of the synthetic oligomers to the 85 bp AluI-XhoI fragment derived from of the BoIFN-$\beta$ subclone plasmids, followed by digestion with EcoRI and XhoI generates a 104 bp fragment flanked by EcoRI and XhoI sticky ends. The entire coding was then assembled into the trp expression vector by ligating the 104 bp fragment together with the approximately 700 bp XhoI-Pst fragment coding for the remainder of each BoIFN-$\beta$ protein and the plasmid pIFN-$\gamma$tr48-13(55) from which the internal EcoRI-PstI fragment had been removed. The resulting plasmids, pBoIFN-$\beta$1trp, pBoIFN-$\beta$2trp and pBoIFN-$\beta$3trp all place the proper transcription and translation of the IFN-$\beta$ genes under the control of the E. coli trp operon.

M. Characterization and Subcloning of Bovine Immune Interferon (BoIFN-$\gamma$) Gene

The ten phage recombinants that hybridized with the human IFN-$\gamma$ probe were purified and DNA was prepared as described above. All ten DNA samples give specific hybridizing bands by Southern blot analysis. Clones $\lambda\gamma$4 and $\lambda\gamma$7 were chosen for further analysis, as they have distinct hybridizing band patterns. Restriction mapping of these two clones shows their DNA sequences overlap with each other. The overlapping region contains the restriction sites XbaI, EcoRV, and NcoI. DNA sequence analysis of these two clones shows an overall similar gene structure to the human immune interferon gene (70) and that $\lambda\gamma$7 contains the sequence coding for the 4th exon and $\lambda\gamma$4 contains sequences coding for the first three exons of bovine IFN-$\gamma$ gene based on DNA sequence homology with the human IFN-$\gamma$ gene. The amino acid sequence deduced for BoIFN-$\gamma$ is compared with that of HuIFN-$\gamma$ (55) and Murine IFN-$\gamma$, in Figure 13.

To assemble the entire bovine IFN-$\gamma$ gene on a continuous segment of DNA, the 3000 bp BamHI-NcoI fragment spanning the first three exons of bovine IFN-$\gamma$ gene derived from $\lambda\gamma$4 and the 2500 bp NcoI-Hind III fragment spanning the last exon derived from $\lambda\gamma$7 were isolated. These two DNA fragments were then cloned into a BamHI-Hind III vector derived from pBR322 via a three part ligation.

N. Expression of Bovine IFN-$\gamma$ Gene in Mammalian System

For purposes of obtaining an intron-less version of BoIFN-$\gamma$ in order that this gene is expressible in a prokaryotic system such as E. coli, the gene was tailored for high level expression in an animal cell expression system to obtain significant quantities of specific mRNA. The 5500 bp BamHI-HindIII fragment spanning the entire bovine IFN-$\gamma$ gene was inserted into a SV40 vector for expression in COS cells (44). Specifically, the BamHI-HindIII bovine IFN-$\gamma$ gene fragment was cloned into a 2800 bp SV40 plasmid vector pDL$\Delta$R1 ((derived from the HBV antigen expression plasmid pHBs348-L by enzymatically deleting the EcoRI site upstream from the SV40 origin of replication selective to the direction of late transcription. Expression plasmid pHBs348-L was constructed by cloning the 1986 base-pair fragment resulting from EcoRI and BglII digestion of HBV (71) (which spans the gene encoding HBsAg) into the plasmid pML (72) at the EcoRI and BamHI sites. (pML is a derivative of pBR322 which has a deletion eliminating sequences which are inhibitory to plasmid replication in monkey cells (72)). The resulting plasmid (pRI-Bg1) was then linearized with EcoRI, and the 348 base-pair fragment representing the SV40 origin region was introduced into the EcoRI site of pRI-Bg1. The origin fragment can insert in either orientation. Since this fragment encodes both the early and late SV40 promoters in addition to the origin of replication, HBV genes could be expressed under the control of either promoter depending on this orientation (pHBs348-L representing HBs expressed under control of the late promoter)) between the BamHI and the Sal I site via a three part ligation in the presence of a 600 bp HindIII-SalI converter fragment derived form pBR322. Transfection of the resultant plasmid into COS cells leads to the efficient expression of bovine IFN-$\gamma$ under the control of SV40 late promotor.

Poly A plus mRNA was prepared from transfected COS cells and used in turn to prepare cDNA by standard procedures (55). cDNA clones hybridizing with bovine IFN-$\gamma$ gene probe were isolated. The cDNA clone with the longest Pst I insert was chosen for further analysis. DNA sequence analysis of this cDNA clone shows all the intron sequences predicted from the bovine IFN-$\gamma$ genomic clone are correctly removed.

The cDNA was tailored for expression in E. coli by the primer repair method described above.

O. Preparation of Bacterial Extracts

Overnight cultures grown in LB broth containing eithe 0.02 mg/ml of ampicillin or 0.005 mg/ml tetracycline were innoculated at a 1:100 dilution into 50 ml of M9 medium (63) containing 0.2 percent glucose, 0.5 percent casamino acids and the appropriate drug, and grown at 37°C with shaking to an A550 = 1.0. Ten ml samples were harvested by centrifugation and immediately quick-frozen in a dry ice-ethanol bath. The frozen pellets were resuspended in 1 ml of 7M guanidine, incubated on ice for 5 minutes, and diluted into PBS for assay. Alternatively, the frozen pellets were lysed by the addition of 0.2 ml of 20 percent sucrose, 100 mM Tris-HCl (pH 8.0), 20 mM EDTA and 5 mg/ml lysozyme. After 20 minutes on ice, 0.8 ml of 0.3 percent Triton X-100, 0.15 M Tris-HCl (pH 8.0), 0.2 M EDTA and 0.1 mM PMSF was added. The lysate was cleared by centrifugation at 19,000 rpm for 15 minutes and the supernatant assayed after dilution into PBS.

P. Interferon Assays

Bovine interferon activity was assayed by a cytopathic effect (CPE) inhibition assay performed in 96 well microtiter plates as follows:

1. Add to each well of a 96 well microliter plate (8 rows x 12 columns) 100 $\mu$l of a suspension of cells in media containing 10 percent fetal calf serum. Cell concentration is adjusted to give confluent monolayer the next day.

2. Rock plates gently on a rocker platform for 10 minutes to evenly distribute cells.

Next Day -

3. Add to each well in the first column 80 $\mu$l of additional media.

4. Add to a well in the first column, 20 $\mu$l of a sample to be assayed for interferon activity.

5. Mix the sample and medium in the well by withdrawing and ejecting 100 $\mu$l of the contents of the well several times with a 100 $\mu$l pipette.

6. Transfer 100 $\mu$l of the contents of a well in the first column horizontally to a well in second column.

7. Mix as in step 3.

8. Continue to transfer 100 $\mu$l of the contents of a well from column to subsequent column until a total of 11 transfers are performed.

9. Remove and discard 100 $\mu$l of the contents of the well in the 12th column. This procedure produces a serial set of two-fold dilutions.

10. Each assay plate includes approprite NIH standards.

11. Incubate plates in a CO$_2$ incubation, 37°C for 24 hours.

12. Each assay plate contains wells which receive 100 $\mu$l of cell suspension and 100 $\mu$l of medium to serve as cell growth controls and wells which receive 100 $\mu$l cell suspension, 100 $\mu$l of medium and 50 $\mu$l of virus suspension to serve as virus-induced cytopathogenic controls.

13. Challenge all wells except cell controls with 50 $\mu$l of a virus suspension. Multiplicity of infection used is that amount of virus which causes 100 percent cytopathic effect on the particular cell line within 24 hours.

14. Reincubate plates for 24 hours at 37°C in CO$_2$ incubation.

15. Remove fluid from plates and stain cells with 0.5 percent crystal violet. Allow cells to stain for 2-5 minutes.

16. Rinse plate well in tap water and allow to dry.

17. Titer of Interferon on sample is the reciprocal of the dilution where 50 percent viable cells remain.

18. The activity of all samples are normalized by the Reference Units Conversion FActor which is calculated from:

$$\frac{\text{Actual Titer of NIH Standard}}{\text{Observed Titer in Assay}} = \text{Reference Units Conversion Factor.}$$

17

(See 69). Extracts prepared from E. coli strain 294 (ATCC No. 31446) transformed with pBoIFN-α1trp55 showed significant activity on a bovine kidney cell line (MDBK) challenged with VS virus (Indiana strain), but not on monkey kidney (VERO), human cervical carcinoma (HeLa), rabbit kidney (Rk-13) or mouse (L929) cell lines challenged in a similar fashion. Control extracts prepared from strain 294 transformed with pBR322 did not exhibit activity on MDBK cells. Table 3 summarizes the in vitro antiviral activity BoIFN-α1 on various challenged animal and human cell lines. BoIFN-α1 is readily distinguished from the human leukocyte IFN's by an apparent lack of antiviral activity on human cells relative to its activity on bovine cells employing VS virus as the challenge. Table 4 shows the level of interferon activity obtained in extracts prepared from E. coli W3110 which has been transformed with the expression plasmids pBoIFN-α4trp15, pBoIFN-β1trp, pBoIFN-β2trp and pBoIFN-β3trp. Particularly significant is the observation that the bovine fibroblast interferons are approximately 30-fold more active on a bovine kidney cell line than on a human amnion cell line, whereas the reciprocal relationship is found for human fibroblast IFN (12).

Table 3

| Cell Line | IFN Preparation | Titer (units/ml) | |
|-----------|-----------------|------|------|
|           |                 | VSV  | EMCV |
| MDBK | LeIF A Standard<br>Bovine leukocyte IFN<br>Control Extract | 640<br>300,000<br><40 | NA<br>NA<br>NA |
| HeLa | LeIF A Standard<br>Bovine leukocyte IFN<br>Control Extract | 650<br><40<br><40 | 1500<br><23<br><23 |
| L-929 | Mouse IFN Standard<br>Bovine leukocyte IFN<br>Control Extract | 640<br><20<br><20 | 1000<br><31<br><31 |
| RK-13 | Rabbit IFN Standard<br>Bovine leukocyte IFN<br>Control Extract | 1000<br><60<br><60 | NA<br>NA<br>NA |
| VERO | LeIF A Standard<br>Bovine leukocyte IFN<br>Control Extract | | 1500<br><12<br><12 |

MDBK = bovine kidney cells VERO = African Green monkey kidney cells HeLa = human cervical carcinoma cells RK-13 = rabbit kidney cells NA = not applicable as virus does not replicate well in respective cell.

Table 4

| Interferon Activity in Extracts of E. coli | | |
|---|---|---|
| E. coli 294 transformed by: | IFN-β activity (units/liter culture) MDBK-VSV | IFN-β activity (units/liter culture) WISH-VSV |
| pIFN-α1 pIFN-β1 pIFN-β2 pIFN-β3 | $1.0 \times 10^8$ $2.2 \times 10^8$ $1.1 \times 10^8$ $6.0 \times 10^8$ | N.D. $6.5 \times 10^6$ $3.5 \times 10^6$ $2.0 \times 10^7$ |
| Bacterial extracts were prepared and assayed for interferon activity using the bovine kidney MDBK cell line and the human amnion WISH cell line and VSV as challenge according to a published procedure (Weck et al., 1981). | | |

Pharmaceutical Compositions

The compounds of the present invention can be formulated according to known methods to prepare pharmaceutically useful compositions, whereby the animal interferon products hereof are combined in admixture with (an) acceptable carrier vehicle(s). Suitable vehicles and their formulation have been described. Such compositions will contain an effective amount of the interferon protein hereof together with a suitable amount of vehicle in order to prepare acceptable compositions suitable for effective administration, via known routes, e.g. parenteral, to the host.

It will be understood that the animal interferons described herein exist with natural allelic variations. These variations may be demonstrated by (an) amino acid difference(s) in the overall sequence or by deletions, substitutions, insertions, inversions or additions of (an) amino acid(s) in said sequence.

Notwithstanding that reference has been made to particular preferred embodiments, it will be further understood that the present invention is not to be construed as limited to such, rather to the lawful scope of the appended claims.

Bibliography

1. Rinaldo et al., Infection and Immunity 14, 660 (1976).
2. Fulton and Rosenquist, Am. J. Vet. Res. 37, 1497 (1976).
3. Babrick and Rouse, Infection and Immunity 13, 1567 (1976).
4. Todd et al., Infection and Immunity 5, 699 (1972).
5. Ahl and Rump, Infection and Immunity 14, 603 (1976).
6. Babrick and Rouse, Intervirology 8, 250 (1977).
7. Tovey et al., J. Gen. Virol. 36, 341 (1977).
8. Goeddel et al., Nature 287, 411 (1980).
9. Goeddel et al., Nature 290, 20 (1981).
10. Yelverton et al., Nucleic Acids Research 9, 731 (1981).
11. Gutterman et al., Annals of Int. Med. 93, 399 (1980).
12. Goeddel et al., Nucleic Acids Reseach 8, 4057 (1980).
13. Yip et al., Proc. Natl. Acad. Sci. (USA) 78, 1601 (1981).
14. Taniguchi et al., Proc. Natl. Acad. Sci. (USA) 78, 3469 (1981).
15. Bloom, Nature 289, 593 (1980).
16. Sonnenfeld et al., Cellular Immunol. 40, 285 (1978).
17. Fleishmann et al., Infection and Immunity 26, 248 (1979).
18. Blalock et al., Cellular Immunology 49, 390 (1980).
19. Rudin et al., Proc. Natl. Acad. Sci. (USA) 77, 5928 (1980).
20. Crane et al., J. Natl. Cancer Inst. 61, 871 (1978).
21. British Patent Publication No. 2007676A.
22. Wetzel, American Scientist 68, 664 (1980).
23. Microbiology, 2nd Edition, Harper and Row Publishers, Inc., Hagerstown, Maryland (1973), esp. pp. 1122 et seq.
24. Scientific American 245, 66 et seq. (1981).

25. British Patent Publication No. 2055382A.

26. German Offenlegungsschrift 2644432.

26a. Leder et al., Science 196, 175 (1977).

27. Chang et al., Nature 275, 617 (1978).

28. Itakura et al., Science 198, 1056 (1977).

29. European Patent Publication No. 0036776.

30. Siebenlist et al., Cell 20, 269 (1980).

31. Stinchcomb et al., Nature 282, 39 (1979).

32. Kingsman et al., Gene 7, 141 (1979).

33. Tschumper et al., Gene 10, 157 (1980).

34. Mortimer et al., Microbiological Reviews 44, 519 (198 ).

35. Miozzari et al., Journal of Bacteriology 134, 48 (1978).

36. Jones, Genetics 85, 23 (1977).

37. Hitzeman, et al., J. Biol. Chem. 255, 12073 (1980).

38. Hess et al., J. Adv. Enzyme Regul. 7, 149 (1968).

39. Holland et al., Biochemistry 17, 4900 (1978).

40. Bostian et al., Proc. Natl. Acad. Sci. (USA) 77, 4504 (1980).

41. The Molecular Biology of Yeast (Aug 11-18, 1981), Cold Spring Harbor Laboratory, Cold Spring Harbor, New York.

42. Chambon, Ann. Rev. Biochemistry, 44, 613 (1975).

43. Tissue Culture, Academic Press, Kruse and Patterson, eds. (1973).

44. Gluzman, Cell 23, 175 (1981).

45. Goeddel et al., Nature 281, 544 (1979).

46. Lusky et al., Nature 293, 79 (1981).

47. Gluzman et al., Cold Spring Harbor Symp. Quant. Biol. 44, 293 (1980).

48. Fiers et al., Nature 273, 113 (1978).

49. Reddy et al., Science 200, 494 (1978).

50. Blin and Stafford Nucleic Acids Research 3, 2303 (1976).

51. Maniatis et al. Cell 15, 687 (1978).

51a. Blattner et al., Science 196, 161 (1977).

52. Rimm et al. Gene 12, 301 (1980).

53. Blattner et al. (1978) Procedures for Use of Charon Phages in Recombinant DNA Research, Research Resources Branch, National Institute of Allergy and Infectious Diseases, Bethesda, Maryland.

54. Blattner et al. Science 202, 1279 (1978).

55. Gray et al., Nature 295, 503 (1982).

56. Southern, J. Mol. Biol. 98, 503 (1975).

57. Weck et al., Nucleic Acids Research 9, 6153.

58. Taylor et al., Biochem. Biophys. Acta 442, 324 (1976).

59. Denhardt, Biochem. Biophys. Res. Comm. 23, 641 (1966).

60. Wahl et al., Proc. Nat. Acad. Sci. 76, 3683 (1979).

61. Nagata et al., Nature 287, 401 (1980).

62. Benton and Davis, Science 196, 180 (1977).

62a. Messing et al., Nucleic Acids Research 9, 309 (1981).

63. Miller (1972) Experiments in Molecular Genetics, Cold Spring Harbor Laboratory, Cold Spring Harbor, New York.

64. Birnboim et al., Nucleic Acids Research 7, 1513 (1979).

65. Maxam and Gilbert, Proc. Nat. Acad. Sci. 74, 560 (1977).

66. McGrath and Levinson, Nature 295, 423 (1982).

67. Itakura et al., Science 198, 1056 (1977).

68. Crea et al., Proc. Natl. Acad. Sci. 75, 5765 (1978).

69. Stewart (1979) The Interferon System, Springer-Verlag, New York, pp. 17 et seq.

70. Gray and Goeddel, Nature 298, 859 (1982).

71. Animal Virus Genetics (Ed. Fields et al.), Chapter 5, p. 57, Academic Press, N.Y. (1980).

72. Lusky and Botchan, Nature 293, 79 (1981).

**Claims**

**Claims for the following Contracting States : BE, CH, DE, FR, GB, IT, LI, LU, NL, SE**

1. A process for identifying DNA encoding an interferon of a non-human animal species, which comprises preparing a library of cloned DNA from said species and probing it with a hybridisation probe of interferon DNA from a different species, and analysing positive clones for interferon-encoding sequences.

2. A process according to claim 1 wherein DNA from said non-human animal species is first separated by electrophoresis and hybridised to said different species interferon DNA under varying hybridisation conditions to determine appropriate hybridisation conditions for probing the library.

3. A process according to claim 1 or claim 2, which further comprises isolating non-human animal interferon-encoding DNA from said positive clones.

4. A process for obtaining DNA encoding a hybrid interferon which comprises identifying a fragment of DNA by the process of any one of claims 1 to 3 which fragment does not encode an entire interferon polypeptide and ligating the fragment with one or more DNA fragments encoding complementary part or parts of an interferon of another member of the same type and of the same animal species and/or of the same or another member of the same type but of a different species.

5. A process which comprises, following the identification of interferon DNA according to any one of claims 1 to 3, producing a non-human animal or hybrid interferon having an amino acid sequence encoded at least in part by said DNA, the process comprising the expression in a recombinant host cell of DNA encoding said interferon product.

6. A process which comprises producing a bovine leukocyte ($\alpha$) interferon comprising an amino acid sequence depicted in any one of Figs. 3a-d hereof by the expression of DNA encoding said interferon in a recombinant host cell transformed with said encoding DNA.

7. A process which comprises producing a bovine fibroblast ($\beta$) interferon comprising an amino acid sequence depicted in any of Figs. 9a-c hereof by the expression of DNA encoding said interferon in a recombinant host cell transformed with said encoding DNA.

8. A process which comprises producing a bovine immune ($\gamma$) interferon comprising an amino acid sequence depicted therefor in Fig. 13 hereof by the expression of DNA encoding said interferon in a recombinant host cell transformed with said encoding DNA.

9. A process according to any one of claims 6 to 8 wherein there is produced a bovine interferon lacking any amino acids before the N-terminal amino acid of the mature sequence.

10. A process according to any one of claims 6 to 8 wherein there is produced a bovine interferon having the methionyl amino acid residue before the N-terminal amino acid of the mature sequence.

11. A process according to any one of claims 6 to 8 wherein there is produced a bovine interferon having conjugated protein before the N-terminal amino acid of the mature sequence.

12. A process according to any one of claims 6 to 8 wherein there is produced a bovine interferon having specifically cleavable conjugated protein before the N-terminal amino acid of the mature sequence.

13. A process which comprises producing a hybrid interferon by the expression of DNA encoding said hybrid in a recombinant host cell transformed with said encoding DNA, the hybrid containing a portion of at least one of the bovine leukocyte interferons depicted in Figs. 3a-d hereof, or a portion of at least one of the bovine fibroblast interferons depicted in Figs. 9a-c hereof, or a portion of the bovine immune interferon depicted in Fig. 13 hereof, with complementary part or parts of an interferon of another member of the same type and of the same animal species and/or of the same or another member of the same type but of a different species.

EP 0 088 622 B1

**14.** A DNA isolate comprising a sequence coding for an interferon referred to in any one of claims 6 to 13.

**15.** An expression vector operably harbouring a DNA sequence coding for an interferon referred to in any one of claims 6 to 13.

**16.** A cell transformed with an expression vector of claim 15.

**17.** A bovine leukocyte interferon comprising an amino acid sequence depicted in any of Figs. 3a-d hereof and devoid of glycosylation.

**18.** A bovine fibroblast interferon comprising an amino acid sequence depicted in any of Figs. 9a-c hereof.

**19.** A bovine immune interferon comprising an amino acid sequence depicted therefor in Fig. 13 hereof.

**20.** A bovine interferon of any of claims 17 to 19 lacking any amino acids before the N-terminal amino acid of the mature sequence.

**21.** A bovine interferon of any of claims 17 to 19 having the methionyl amino acid residue before the N-terminal amino acid of the mature sequence.

**22.** A bovine interferon of any of claims 17 to 19 having conjugated protein before the N-terminal amino acid of the mature sequence.

**23.** A bovine interferon of any of claims 17 to 19 having specifically cleavable conjugated protein before the N-terminal amino acid of the mature sequence.

**24.** A hybrid interferon containing a portion of at least one of the bovine leukocyte interferons depicted in Figs. 3a-d hereof, or a portion of at least one of the bovine fibroblast interferons depicted in Figs. 9a-c hereof, or a portion of the bovine immune interferon depicted in Fig. 13 hereof, with complementary part or parts of an interferon of another member of the same type and of the same animal species and/or of the same or another member of the same type but of a different species.

**Claims for the following Contracting State : AT**

**1.** A process for identifying DNA encoding an interferon of a non-human animal species, which comprises preparing a library of cloned DNA from said species and probing it with a hybridisation probe of interferon DNA from a different species, and analysing positive clones for interferon-encoding sequences.

**2.** A process according to claim 1 wherein DNA from said non-human animal species is first separated by electrophoresis and hybridised to said different species interferon DNA under varying hybridisation conditions to determine appropriate hybridisation conditions for probing the library.

**3.** A process according to claim 1 or claim 2, which further comprises isolating non-human animal interferon-encoding DNA from said positive clones.

**4.** A process for obtaining DNA encoding a hybrid interferon which comprises identifying a fragment of DNA by the process of any one of claims 1 to 3 which fragment does not encode an entire interferon polypeptide and ligating the fragment with one or more DNA fragments encoding complementary part or parts of an interferon of another member of the same type and of the same animal species and/or of the same or another member of the same type but of a different species.

**5.** A process which comprises, following the identification of interferon DNA according to any one of claims 1 to 3, producing a non-human animal or hybrid interferon having an amino acid sequence encoded at least in part by said DNA, the process comprising the expression in a recombinant host cell of DNA encoding said interferon product.

22

**6.** A process which comprises producing a bovine leukocyte (α) interferon comprising an amino acid sequence depicted in any one of Figs. 3a-d hereof by the expression of DNA encoding said interferon in a recombinant host cell transformed with said encoding DNA.

**7.** A process which comprises producing a bovine fibroblast (β) interferon comprising an amino acid sequence depicted in any of Figs. 9a-c hereof by the expression of DNA encoding said interferon in a recombinant host cell transformed with said encoding DNA.

**8.** A process which comprises producing a bovine immune (γ) interferon comprising an amino acid sequence depicted therefor in Fig. 13 hereof by the expression of DNA encoding said interferon in a recombinant host cell transformed with said encoding DNA.

**9.** A process according to any one of claims 6 to 8 wherein there is produced a bovine interferon lacking any amino acids before the N-terminal amino acid of the mature sequence.

**10.** A process according to any one of claims 6 to 8 wherein there is produced a bovine interferon having the methionyl amino acid residue before the N-terminal amino acid of the mature sequence.

**11.** A process according to any one of claims 6 to 8 wherein there is produced a bovine interferon having conjugated protein before the N-terminal amino acid of the mature sequence.

**12.** A process according to any one of claims 6 to 8 wherein there is produced a bovine interferon having specifically cleavable conjugated protein before the N-terminal amino acid of the mature sequence.

**13.** A process which comprises producing a hybrid interferon by the expression of DNA encoding said hybrid in a recombinant host cell transformed with said encoding DNA, the hybrid containing a portion of at least one of the bovine leukocyte interferons depicted in Figs. 3a-d hereof, or a portion of at least one of the bovine fibroblast interferons depicted in Figs. 9a-c hereof, or a portion of the bovine immune interferon depicted in Fig. 13 hereof, with complementary part or parts of an interferon of another member of the same type and of the same animal species and/or of the same or another member of the same type but of a different species.

**14.** A cell transformed with an expression vector operably harbouring a DNA sequence coding for an interferon of any one of claims 6 to 13.

**Patentansprüche**
**Patentansprüche für folgende Vertragsstaaten : BE, CH, DE, FR, GB, IT, LI, LU, NL, SE**

**1.** Verfahren zum Identifizieren von DNA, die für ein Interferon einer nicht-menschlichen Tierart kodiert, das das Herstellen einer Sammlung von klonierter DNA aus dieser Art und deren Sondieren mit einer Hybridisierungs-Sonde aus Interferon-DNA aus einer anderen Art und das Analysieren positiver Klons auf für Interferon kodierende Sequenzen umfaßt.

**2.** Verfahren nach Anspruch 1, worin DNA aus der nicht-menschlichen Tierart zuerst mittels Elektrophorese getrennt und unter variierenden Hybridisierungs-Bedingungen an die Interferon-DNA der anderen Art hybridisiert wird, um die geeigneten Hybridisierungs-Bedingungen zum Sondieren der Sammlung zu bestimmen.

**3.** Verfahren nach Anspruch 1 oder 2, das weiters das Isolieren von für Interferon kodierender nicht-menschlicher Tier-DNA aus den positiven Klons umfaßt.

**4.** Verfahren zur Gewinnung von DNA, die für ein Hybrid-Interferon kodiert, umfassend das Identifizieren eines DNA-Fragments nach dem Verfahren nach irgendeinem der Ansprüche 1 - 3, wobei das Fragment nicht für ein vollständiges Interferon-Polypeptid kodiert, und das Ligieren des Fragments mit einem oder mehreren DNA-Fragmenten, die für einen komplementären Teil oder mehrere komplementäre Teile eines Interferons eines anderen Vertreters desselben Typs und derselben Tierart und/oder desselben oder eines anderen Vertreters desselben Typs jedoch einer anderen Art kodiert oder kodieren.

**5.** Verfahren, umfassend nach der Identifizierung von Interferon-DNA gemäß irgendeinem der Ansprüche 1 - 3, das Produzieren eines nicht-menschlichen Tier- oder Hybrid-Interferons mit einer Aminosäuresequenz, für die zumindest teilweise von dieser DNA kodiert wird, und das Exprimieren von DNA, die für das Interferon-Produkt kodiert, in einer rekombinanten Wirtszelle.

**6.** Verfahren, umfassend das Produzieren eines Rinder-Leukozyten-α-Interferons, das eine in einer der Figuren 3a-d dargestellte Aminosäuresequenz umfaßt, durch das Exprimieren von für das Interferon kodierender DNA in einer mit der kodierenden DNA transformierten, rekombinanten Wirtszelle.

**7.** Verfahren, umfassend das Produzieren eines Rinder-Fibroblasten-β-Interferons, das eine in einer der Figuren 9a-c dargestellte Aminosäuresequenz umfaßt, durch das Exprimieren von für das Interferon kodierender DNA in einer mit der kodierenden DNA transformierten, rekombinanten Wirtszelle.

**8.** Verfahren, umfassend das Produzieren eines Rinder-Immun-γ-Interferons, das eine dafür in Figur 13 dargestellte Aminosäuresequenz umfaßt, durch das Exprimieren von für das Interferon kodierender DNA in einer mit der kodierenden DNA transformierten, rekombinanten Wirtszelle.

**9.** Verfahren nach irgendeinem der Ansprüche 6 - 8, worin ein Rinder-Interferon produziert wird, dem alle Aminosäuren vor der N-terminalen Aminosäure der reifen Sequenz fehlen.

**10.** Verfahren nach irgendeinem der Ansprüche 6 - 8, worin ein Rinder-Interferon produziert wird, das den Methionyl-Aminosäurerest vor der N-terminalen Aminosäure der reifen Sequenz aufweist.

**11.** Verfahren nach irgendeinem der Ansprüche 6 - 8, worin ein Rinder-Interferon produziert wird, das konjugiertes Protein vor der N-terminalen Aminosäure der reifen Sequenz aufweist.

**12.** Verfahren nach irgendeinem der Ansprüche 6 - 8, worin ein Rinder-Interferon produziert wird, das spezifisch spaltbares, konjugiertes Protein vor der N-terminalen Aminosäure der reifen Sequenz aufweist.

**13.** Verfahren, umfassend das Produzieren eines Hybrid-Interferons durch Exprimieren von für das Hybrid kodierender DNA in einer mit der kodierenden DNA transformierten, rekombinanten Wirtszelle, wobei das Hybrid einen Abschnitt zumindest eines der in den Figuren 3a-d dargestellten Rinder-Leukozyten-Interferone oder einen Abschnitt zumindest eines der in den Figuren 9a-c dargestellten Rinder-Fibroblasten-Interferone oder einen Abschnitt des in Figur 13 dargestellten Rinder-Immun-Interferons und einen komplementären Teil oder mehrere komplementäre Teile eines Interferons eines anderen Vertreters desselben Typs und derselben Tierart und/oder desselben oder eines anderen Vertreters desselben Typs jedoch einer anderen Art enthält.

**14.** DNA-Isolat, umfassend eine für ein Interferon nach irgendeinem der Ansprüche 6 - 13 kodierende Sequenz.

**15.** Expressionsvektor, der eine für ein Interferon nach irgendeinem der Ansprüche 6 - 13 kodierende DNA-Sequenz operabel enthält.

**16.** Zelle, die mit einem Expressionsvektor nach Anspruch 15 transformiert ist.

**17.** Rinder-Leukozyten-Interferon, das eine in einer der Figuren 3a-d dargestellte Aminosäuresequenz ohne Glykosylierung umfaßt.

**18.** Rinder-Fibroblasten-Interferon, das eine in einer der Figuren 9a-c dargestellte Aminosäuresequenz umfaßt.

**19.** Rinder-Immun-Interferon, das eine dafür in Figur 13 dargestellte Aminosäuresequenz umfaßt.

**20.** Rinder-Interferon nach irgendeinem der Ansprüche 17 - 19, dem alle Aminosäuren vor der N-terminalen Aminosäure der reifen Sequenz fehlen.

**21.** Rinder-Interferon nach irgendeinem der Ansprüche 17 - 19, das den Methionyl-Aminosäurerest vor der N-terminalen Aminosäure der reifen Sequenz aufweist.

**22.** Rinder-Interferon nach irgendeinem der Ansprüche 17 - 19, das konjugiertes Protein vor der N-terminalen Aminosäure der reifen Sequenz aufweist.

**23.** Rinder-Interferon nach irgendeinem der Ansprüche 17 - 19, das spezifisch spaltbares, konjugiertes Protein vor der N-terminalen Aminosäure der reifen Sequenz aufweist.

**24.** Hybrid-Interferon, das einen Abschnitt zumindest eines der in den Figuren 3a-d dargestellten Rinder-Leukozyten-Interferone oder einen Abschnitt zumindest eines der in den Figuren 9a-c dargestellten Rinder-Fibroblasten-Interferone oder einen Abschnitt des in Figur 13 dargestellten Rinder-Immun-Interferons und einen komplementären Teil oder mehrere komplementäre Teile eines Interferons eines anderen Vertreters desselben Typs und derselben Tierart und/oder desselben oder eines anderen Vertreters desselben Typs jedoch einer anderen Art enthält.

**Patentansprüche für folgenden Vertragsstaat : AT**

**1.** Verfahren zum Identifizieren von DNA, die für ein Interferon einer nicht-menschlichen Tierart kodiert, das das Herstellen einer Sammlung von klonierter DNA aus dieser Art und deren Sondieren mit einer Hybridisierungs-Sonde aus Interferon-DNA aus einer anderen Art und das Analysieren positiver Klons auf für Interferon kodierende Sequenzen umfaßt.

**2.** Verfahren nach Anspruch 1, worin DNA aus der nicht-menschlichen Tierart zuerst mittels Elektrophorese getrennt und unter variierenden Hybridisierungs-Bedingungen an die Interferon-DNA der anderen Art hybridisiert wird, um die geeigneten Hybridisierungs-Bedingungen zum Sondieren der Sammlung zu bestimmen.

**3.** Verfahren nach Anspruch 1 oder 2, das weiters das Isolieren von für Interferon kodierender nicht-menschlicher Tier-DNA aus den positiven Klons umfaßt.

**4.** Verfahren zur Gewinnung von für ein Hybrid-Interferon kodierender DNA, umfassend das Identifizieren eines DNA-Fragments nach dem Verfahren nach irgendeinem der Ansprüche 1 - 3, wobei das Fragment nicht für ein vollständiges Interferon-Polypeptid kodiert, und das Ligieren des Fragments mit einem oder mehreren DNA-Fragmenten, die für einen komplementären Teil oder mehrere komplementäre Teile eines Interferons eines anderen Vertreters desselben Typs und derselben Tierart und/oder desselben oder eines anderen Vertreters desselben Typs jedoch einer anderen Art kodiert oder kodieren.

**5.** Verfahren, umfassend nach der Identifizierung von Interferon-DNA gemäß irgendeinem der Ansprüche 1 - 3, das Produzieren eines nicht-menschlichen Tier- oder Hybrid-Interferons mit einer Aminosäuresequenz, für die zumindest teilweise von dieser DNA kodiert wird, und das Exprimieren von DNA, die für das Interferon-Produkt kodiert, in einer rekombinanten Wirtszelle.

**6.** Verfahren, umfassend das Produzieren eines Rinder-Leukozyten-$\alpha$-Interferons, das eine in einer der Figuren 3a-d dargestellte Aminosäuresequenz umfaßt, durch das Exprimieren von für das Interferon kodierender DNA in einer mit der kodierenden DNA transformierten, rekombinanten Wirtszelle.

**7.** Verfahren, umfassend das Produzieren eines Rinder-Fibroblasten-$\beta$-Interferons, das eine in einer der Figuren 9a-c dargestellte Aminosäuresequenz umfaßt, durch das Exprimieren von für das Interferon kodierender DNA in einer mit der kodierenden DNA transformierten, rekombinanten Wirtszelle.

**8.** Verfahren, umfassend das Produzieren eines Rinder-Immun-$\gamma$-Interferons, das eine dafür in Figur 13 dargestellte Aminosäuresequenz umfaßt, durch das Exprimieren von für das Interferon kodierender DNA in einer mit der kodierenden DNA transformierten, rekombinanten Wirtszelle.

**9.** Verfahren nach irgendeinem der Ansprüche 6 - 8, worin ein Rinder-Interferon produziert wird, dem alle Aminosäuren vor der N-terminalen Aminosäure der reifen Sequenz fehlen.

**10.** Verfahren nach irgendeinem der Ansprüche 6 - 8, worin ein Rinder-Interferon produziert wird, das den Methionyl-Aminosäurerest vor der N-terminalen Aminosäure der reifen Sequenz aufweist.

**11.** Verfahren nach irgendeinem der Ansprüche 6 - 8, worin ein Rinder-Interferon produziert wird, das konjugiertes Protein vor der N-terminalen Aminosäure der reifen Sequenz aufweist.

**12.** Verfahren nach irgendeinem der Ansprüche 6 - 8, worin ein Rinder-Interferon produziert wird, das spezifisch spaltbares, konjugiertes Protein vor der N-terminalen Aminosäure der reifen Sequenz aufweist.

**13.** Verfahren, umfassend das Produzieren eines Hybrid-Interferons durch Exprimieren von für das Hybrid kodierender DNA in einer mit der kodierenden DNA transformierten, rekombinanten Wirtszelle, wobei das Hybrid einen Abschnitt zumindest eines der in den Figuren 3a-d dargestellten Rinder-Leukozyten-Interferone oder einen Abschnitt zumindest eines der in den Figuren 9a-c dargestellten Rinder-Fibroblasten-Interferone oder einen Abschnitt des in Figur 13 dargestellten Rinder-Immun-Interferons und einen komplementären Teil oder mehrere komplementäre Teile eines Interferons eines anderen Vertreters desselben Typs und derselben Tierart und/oder desselben oder eines anderen Vertreters desselben Typs jedoch einer anderen Art enthält.

**14.** Zelle, die mit einem Expressionsvektor transformiert ist, der eine für ein Interferon nach irgendeinem der Ansprüche 6 - 13 kodierende DNA operabel enthält.

**Revendications**
**Revendications pour les Etats contractants suivants : BE, CH, DE, FR, GB, IT, LI, LU, NL, SE**

**1.** Procédé d'identification de l'ADN codant pour un interféron d'une espèce animale autre que l'homme, qui comprend la préparation d'une banque d'ADN cloné provenant de ladite espèce et le sondage de cette banque avec une sonde d'hybridation d'ADN d'interféron provenant d'une espèce différente, et l'analyse des clones positifs pour les séquences codant pour l'interféron.

**2.** Procédé suivant la revendication 1, dans lequel l'ADN provenant de l'espèce animale autre que l'homme est tout d'abord séparé par électrophorèse et hybridé à l'ADN d'interféron d'une espèce différente dans des conditions d'hybridation variables pour déterminer les conditions d'hybridation appropriées pour le sondage de la banque.

**3.** Procédé suivant la revendication 1 ou la revendication 2, qui comprend en outre l'isolement de l'ADN codant pour l'interféron d'une espèce animale autre que l'homme des clones positifs.

**4.** Procédé d'obtention de l'ADN codant pour un interféron hybride, qui comprend l'identification d'un fragment d'ADN par le procédé suivant l'une quelconque des revendications 1 à 3, fragment qui ne code pas pour un polypeptide d'interféron complet, et la ligation du fragment à un ou plusieurs fragments d'ADN codant pour une ou plusieurs parties complémentaires d'un interféron d'un autre membre du même type et de la même espèce animale et/ou du même membre ou d'un autre membre du même type mais d'une espèce différente.

**5.** Procédé qui comprend, après l'identification de l'ADN d'interféron suivant l'une quelconque des revendications 1 à 3, la production d'un interféron d'un animal autre que l'homme ou interféron hybride ayant une séquence d'aminoacides codée au moins en partie par ledit ADN, procédé comprenant l'expression dans une cellule hôte recombinante de l'ADN codant pour ledit produit consistant en interféron.

**6.** Procédé qui comprend la production d'un interféron ($\alpha$) leucocytaire bovin comprenant une séquence d'aminoacides représentée sur l'une quelconque des figures 3a à 3d par expression de l'ADN codant pour ledit interféron dans une cellule hôte recombinante transformée avec ledit ADN codant.

**7.** Procédé qui comprend la production d'un interféron ($\beta$) de fibroblastes bovins, comprenant une séquence d'aminoacides représentée sur l'une quelconque des figures 9a à 9c, par expression de l'ADN codant pour ledit interféron dans une cellule hôte recombinante transformée avec ledit ADN codant.

8.  Procédé qui comprend la production d'un interféron (γ) immunitaire bovin, comprenant une séquence d'aminoacides représentée sur la figure 13, par expression de l'ADN codant pour ledit interféron dans une cellule hôte recombinante transformée avec ledit ADN codant.

9.  Procédé suivant l'une quelconque des revendications 6 à 8, dans lequel est produit un interféron bovin dépourvu de tous les aminoacides avant l'aminoacide N-terminal de la séquence mature.

10. Procédé suivant l'une quelconque des revendications 6 à 8, dans lequel est produit un interféron bovin possédant le résidu d'aminoacide méthionyle avant l'aminoacide N-terminal de la séquence mature.

11. Procédé suivant l'une quelconque des revendications 6 à 8, dans lequel est produit un interféron bovin possédant une protéine conjuguée avant l'aminoacide N-terminal de la séquence mature.

12. Procédé suivant l'une quelconque des revendications 6 à 8, dans lequel est produit un interféron bovin possédant une protéine conjuguée, clivable spécifiquement, avant l'aminoacide N-terminal de la séquence mature.

13. Procédé qui comprend la production d'un interféron hybride par expression de l'ADN codant pour ledit hydride dans une cellule hôte recombinante transformée avec ledit ADN codant, l'hybride contenant une portion d'au moins un des interférons leucocytaires bovins représentés sur les figures 3a à 3d, ou une portion d'au moins un des interférons de fibroblastes bovins représentés sur les figures 9a à 9c, ou bien une portion de l'interféron immunitaire bovin représenté sur la figure 13, avec une ou plusieurs parties complémentaires d'un interféron d'un autre membre du même type et de la même espèce animale et/ou du même membre ou d'un autre membre du même type mais d'une espèce différente.

14. Isolat d'ADN comprenant une séquence codant pour l'interféron mentionné dans l'une quelconque des revendications 6 à 13.

15. Vecteur d'expression portant de manière fonctionnelle une séquence d'ADN codant pour un interféron mentionné dans l'une quelconque des revendications 6 à 13.

16. Cellule transformée avec un vecteur d'expression suivant la revendication 15.

17. Interféron leucocytaire bovin comprenant une séquence d'aminoacides représentée sur l'une quelconque des figures 3a à 3d et dépourvu de glycosylation.

18. Interféron de fibroblastes bovins comprenant une séquence d'aminoacides représentée sur l'une quelconque des figures 9a à 9c.

19. Interféron immunitaire bovin comprenant une séquence d'aminoacides représentée sur la figure 13.

20. Interféron bovin suivant l'une quelconque des revendications 17 à 19, dépourvu de n'importe quels aminoacides avant l'aminoacide N-terminal de la séquence mature.

21. Interféron bovin suivant l'une quelconque des revendications 17 à 19, possédant le résidu d'aminoacide méthionyle avant l'aminoacide N-terminal de la séquence mature.

22. Interféron bovin suivant l'une quelconque des revendications 17 à 19, possédant une protéine conjuguée avant l'aminoacide N-terminal de la séquence mature.

23. Interféron bovin suivant l'une quelconque des revendications 17 à 19, possédant une protéine conjuguée, clivable spécifiquement, avant l'aminoacide N-terminal de la séquence mature.

24. Interféron hybride contenant une portion d'au moins un des interférons leucocytaires bovins représentés sur les figures 3a à 3d, ou une portion d'au moins des interférons de fibroblastes bovins représentés sur les figures 9a à 9c, ou bien une portion de l'interféron immunitaire bovin représenté sur la figure 13, avec une ou plusieurs parties complémentaires d'un interféron d'un autre membre du même type et de la même espèce animale et/ou du même membre ou d'un autre membre du même type mais d'une espèce différente.

**Revendications pour l'Etat contractant suivant : AT**

1.   Procédé d'identification de l'ADN codant pour un interféron d'une espèce animale autre que l'homme, qui comprend la préparation d'une banque d'ADN cloné provenant de ladite espèce et le sondage de cette banque avec une sonde d'hybridation d'ADN d'interféron d'une espèce différente, et l'analyse des clones positifs pour les séquences codant pour l'interféron.

2.   Procédé suivant la revendication 1, dans lequel l'ADN de l'espèce animale autre que l'homme est tout d'abord séparé par électrophorèse et hybridé à l'ADN d'interféron d'une espèce différente dans des conditions d'hybridation variables pour déterminer les conditions d'hybridation appropriées pour le sondage de la banque.

3.   Procédé suivant la revendication 1 ou la revendication 2, qui comprend en outre l'isolement de l'ADN codant pour l'interféron d'une espèce animale autre que l'homme à partir des clones positifs.

4.   Procédé d'obtention de l'ADN codant pour un interféron hybride, qui comprend l'identification d'un fragment d'ADN par le procédé suivant l'une quelconque des revendications 1 à 3, fragment qui ne code pas pour un polypeptide d'interféron complet, et la ligation du fragment à un ou plusieurs fragments d'ADN codant pour une ou plusieurs parties complémentaires d'un interféron d'un autre membre du même type et de la même espèce animale et/ou du même membre ou d'un autre membre du même type mais d'une espèce différente.

5.   Procédé qui comprend, après l'identification de l'ADN d'interféron suivant l'une quelconque des revendications 1 à 3, la production d'un interféron d'une espèce animale autre que l'homme ou interféron hybride ayant une séquence d'aminoacides codée au moins en partie par ledit ADN, procédé comprenant l'expression dans une cellule hôte recombinante de l'ADN codant pour ledit produit consistant en un interféron.

6.   Procédé qui comprend la production d'un interféron ($\alpha$) leucocytaire bovin comprenant une séquence d'aminoacides représentée sur l'une quelconque des figures 3a à 3d par expression de l'ADN codant pour ledit interféron dans une cellule hôte recombinante transformée avec ledit ADN codant.

7.   Procédé qui comprend la production d'un interféron ($\beta$) de fibroblastes bovins, comprenant une séquence d'aminoacides représentée sur l'une quelconque des figures 9a à 9c, par expression de l'ADN codant pour ledit interféron dans une cellule hôte recombinante transformée avec ledit ADN codant.

8.   Procédé qui comprend la production d'un interféron ($\gamma$) immunitaire bovin, comprenant une séquence d'aminoacides représentée sur la figure 13, par expression de l'ADN codant pour ledit interféron dans une cellule hôte recombinante transformée avec ledit ADN codant.

9.   Procédé suivant l'une quelconque des revendications 6 à 8, dans lequel est produit un interféron bovin dépourvu de tous les aminoacides avant l'aminoacide N-terminal de la séquence mature.

10.   Procédé suivant l'une quelconque des revendications 6 à 8, dans lequel est produit un interféron bovin possédant le résidu d'aminoacide méthionyle avant l'aminoacide N-terminal de la séquence mature.

11.   Procédé suivant l'une quelconque des revendications 6 à 8, dans lequel est produit un interféron bovin possédant une protéine conjuguée avant l'aminoacide N-terminal de la séquence mature.

12.   Procédé suivant l'une quelconque des revendications 6 à 8, dans lequel est produit un interféron bovin possédant une protéine conjuguée, clivable spécifiquement, avant l'aminoacide N-terminal de la séquence mature.

28

**13.** Procédé qui comprend la production d'un interféron hybride par expression de l'ADN codant pour ledit hydride dans une cellule hôte recombinante transformée avec ledit ADN codant, l'hybride contenant une portion d'au moins un des interférons leucocytaires bovins représentés sur les figures 3a à 3d, ou une portion d'au moins un des interférons de fibroblastes bovins représentés sur les figures 9a à 9c, ou bien une portion de l'interféron immunitaire bovin représenté sur la figure 13, avec une ou plusieurs parties complémentaires d'un interféron d'un autre membre du même type et de la même espèce animale et/ou du même membre ou d'un autre membre du même type mais d'une espèce différente.

**14.** Cellule transformée avec un vecteur d'expression portant de manière fonctionnelle une séquence codant pour un interféron suivant l'une quelconque des revendications 6 à 13.

formamide concentration

Fig.1

Fig.2

```
GGATCCACAGCATAAATGTGTTGTCACAATTTCACGGTGGGGGTAATTAGGAAAAAAAAAATCTCAGAAGAACTGTCAATAGGGGAAGGGGGGGCAATAATGAAAACAACGTTTGCGAA
                              50                                                              100

ATGCTGTCCTAACCCATTTGAAGAGTACAAACTGAAAAACAAAAACAAAAGTAGAAAGCAAGAGGGAACTTTCAGAAAATGGAAACCATGGACTCCTATTTAAGACACAGACCTGAAGG
                           150                                                           200

                                                                                 S1                                  S10
                                                                                 met ala pro ala trp ser leu leu leu ala
AAGGTCTTCAGAGAACCTAGAAAGCAGGTTCACAGAGTCACCCACCGCCCCAGGCCACAAGCATCTTCAAGGTCCCCG  ATG GCC CCA GCC TGG TCC CTC CTC CTG GCT
        250                                        300

                                              S20        S23   1                                        10
leu leu leu leu ser cys asn ala ile cys ser leu gly CYS HIS LEU PRO HIS SER HIS SER LEU ALA LYS ARG ARG VAL LEU THR LEU
CTG CTG CTG CTC AGC TGC AAC GCC ATC TGC TCT CTG GGC TGC CAC CTG CCT CAC TCC CAC AGC CTG GCC AAG AGG AGA GTC CTG ACA CTC
   350                                                        400

           20                                        30                                        40
LEU ARG GLN LEU ARG ARG VAL SER PRO SER SER CYS LEU GLN ASP ARG ASN ASP PHE ALA PHE PRO GLN GLU ALA LEU GLY GLY SER GLN
CTG CGA CAA CTG AGG AGG GTC TCC CCT TCC TCC TGC CTG CAG GAC AGA AAT GAC TTC GCA TTC CCC CAG GAG GCG CTG GGT GGC AGC CAG
              450                                                        500

           50                                        60                                        70
LEU GLN LYS ALA GLN ALA ILE SER VAL LEU HIS GLU VAL THR GLN HIS THR PHE GLN LEU PHE SER THR GLU GLY SER ALA ALA VAL TRP
TTG CAG AAG GCT CAA GCC ATC TCT GTA CTC CAC GAG GTG ACC CAA CAC ACC TTC CAG CTT TTC AGC ACA GAG GGC TCG GCC GCT GTG TGG
                 550                                                        600

           80                                        90                                        100
ASP GLU SER LEU LEU ASP LYS LEU ARG THR ALA LEU ASP GLN GLN LEU THR ASP LEU GLN ALA CYS LEU ARG GLN GLU GLU GLY LEU PRO
GAT GAG AGC CTC CTG GAC AAG CTC CGC ACT GCA CTG GAT CAG CAG CTC ACT GAC CTG CAA GCC TGT CTG AGG CAG GAG GAG GGG CTG CCA
                             650                                                        700

           110                                       120                                       130
GLY ALA PRO LEU LEU LYS GLU ASP SER SER LEU ALA VAL ARG LYS TYR PHE HIS ARG LEU THR LEU TYR LEU GLN GLU LYS ARG HIS SER
GGG GCT CCC CTG CTC AAG GAG GAC TCC AGC CTG GCT GTG AGG AAA TAC TTC CAC AGA CTC ACT CTC TAT CTG CAA GAG AAG AGA CAC AGC
                                                  750

           140                                       150                                       160            166Stop
PRO CYS ALA TRP GLU VAL VAL ARG ALA GLN VAL MET ARG ALA PHE SER SER SER THR ASN LEU GLN GLU ARG PHE ARG ARG LYS ASP OP
CCT TGT GCC TGG GAG GTT GTC AGA GCA CAA GTC ATG AGA GCC TTC TCT TCC TCA ACA AAC TTG CAG GAG AGA TTC AGG AGA AAG GAC TGA
   800                                                        850

CACACACCTGGTTCAACACGGAAATGATTCTCACGGACCAACAGACCACACTTCCTCCTGCGCTGCCATGTGGAAGACTCATTTCTGCTGTCATCAGGCACTGAACTGAATCAATTTGTT
         900                                            950                                                  1000

AAATGATTTCAGGTATATTATGTGACATCATGATCTACTCTACAGGCACTACTCTGTCCCAGATACTCAAGCTAATCCATCTACTTATTTATCTATTTGGTATTTATTTATCTAATTTAA
                       1050                                               1100

TATTTATTTATCTATATATAAAGAATTAAATTATTTTGTTCATATAATTATGTATGTATAATTAATGGAAAAATATATTTTGTATTTAGTCAATTTATGAGTTTTCTTCATTCATTAAAC
              1150                                               1200

CTTACTATAAAAATCTTCCTTTGTTTTTCTTTAAAAAAGAAACATGAAGACTGAATATGCAACTTGATTAAAGAATGCATTTTTATAATTCCTTCACCCATTTTTGTGATTTGCACATTA
1250                                              1300                                              1350

CAAATGGGGATTTTGGGGGGGATTTTCTGACCGGAACTTGGAAGCGACGAACCTGAAAGAAGGACACTCAGACAGTCTCTTGCAAGGACTGACAAGTTTATTTC
                   1400                                           1450
```

# Fig.3a

32

BoIFN-α2

GAAAAAAATATCTAAAAGGCTCTGTGGGCAGAAGAAAGAAGAGCAACATGAAAAAAAAAAATGATTGGGTAGTGCAGCCC

TAACCCACTGGAGAGTGCAAACTGAAAAGCAAAAACAAAAGTAGAAAATAAGAGGGAACTTTCACAAAGTGGAAACCAT

GGGCTCCTATTTAAGACACAGGCCTGAAGGAAGGTCTTCAGAGAATCTAGAGAGCAGGTTCACAGAGTCACCCACTGCC

```
                                     S1
                                     met ala pro ala trp ser phe leu leu ala leu leu
CCAGGGCAGAAGCATCTGCAAGGTCCCCG        ATG GCC CCA GCC TGG TCC TTC CTC CTG GCC CTG CTG

                       S20         S23   1
leu leu ser cys asn ala ile cys ser leu gly cys his leu pro his thr his ser leu
CTG CTC AGC TGC AAC GCC ATC TGC TCT TTG GGT TGC CAC CTG CCT CAC ACC CAC AGC CTG

 10                                  20
pro asn arg arg val leu thr leu leu arg gln leu arg arg val ser pro ser ser cys
CCC AAC AGG AGG GTC CTG ACA CTC CTG CGA CAA CTG AGG AGG GTC TCC CCT TCC TCC TGC

 30                                  40
leu gln asp arg asn asp phe ala phe pro gln glu ala leu gly gly ser gln leu gln
CTG CAG GAC AGA AAT GAC TTT GCA TTC CCC CAG GAG GCG CTG GGT GGC AGC CAG TTG CAG

 50                                  60
lys ala gln ala ile ser val leu his glu val thr gln his thr phe gln leu phe ser
AAG GCT CAA GCC ATC TCT GTG CTC CAC GAG GTG ACC CAG CAC ACC TTC CAG CTC TTC AGC

 70                                  80
thr glu gly ser ala ala val trp asp gln ser leu leu asp lys leu arg ala ala leu
ACA GAG GGC TCG GCC GCT GTG TGG GAC CAG AGC CTC CTG GAC AAG CTC CGA GCT GCA CTG

 90                                  100
asp gln gln leu thr asp leu gln ala cys leu arg gln glu glu gly leu arg gly ala
GAT CAG CAG CTC ACT GAC CTG CAA GCC TGT CTG AGG CAG GAG GAG GGG CTG CGA GGG GCT

110                                  120
pro leu leu lys glu asp ala ser leu ala val arg lys tyr phe his arg leu thr leu
CCC CTG CTC AAG GAG GAT GCC AGC CTG GCT GTG AGG AAA TAC TTC CAC AGA CTC ACT CTC

130                                  140
tyr leu gln glu lys arg his ser pro cys ala trp glu val val arg ala glu val met
TAT CTG CAA GAG AAG AGA CAC AGC CCT TGT GCC TGG GAG GTT GTC AGA GCA GAA GTC ATG

150                                  160                        166
arg ala phe ser ser ser thr asn leu gln glu lys phe arg arg lys asp OP
AGA GCC TTC TCT TCT TCA ACA AAC TTG CAG GAG AAA TTC AGG AGA AAG GAC TGA CACACA
```

CCTGGTTCAACACGGAAATGATTCTCATGGACCAACAGACCACACTTCCTCCTGCACTGCCATGTGGAAGACTCTCAT

TTCTGCTGTCATTGCACCCTGAAATGAATCAATATGTCAAATGATTTCTGGAATATTAAGTAACATCATGTTCTACTC

TATAGGCAAAACAGATGCCGAAGCTCATCTATCTACATATTTAACTACTTGGACATTTATTTATTTATTTTAATATTT

ATTTAACTATTTATAAATATTTAAATTATTTTGTTGATAAAGTATTATGTATGTACATTTAGGGGAAAATGTATATTT

TGTATTTAGTCAGTTTATGATTTTTCTTCCTTTATTAAATTTTTACTGTAAAAGACTTACTTTGTTTTTTCGTTAAAA

CAGAGCCACCAAGCCTGAATGGCAGCTTGATTAAAAATTGCATTTTATGATTCCTTGAGCCCTTTTTAGGATCTGCAT

GTTAGAAGTAAAAATACTCTAGCTCTAGCTATGTTTTTTGTTGCTCTGAGGACCTTGAAGGGAACATAACCACTCCAG

TGCTTTTTGTAACTCTGATTTTTTTTTTCAAAAAAAAAGTAACCTAAAAACAACCATCAAAAAAAAAAATCCATGCTTCAG

GATTTGATGAATTC

# Fig.3b

BoIFN-α3

GGATCCACAGCATAAATGTGTTGTCACAATTTCACGGTGGGGGGTAATTAGTAAAAAAAAAAAATTTCAGAAGACTCTGTC

AATAGGGGAAGGGGGGTCAATAATGAAAACAACGTTTGCAAAATGCTGTCCTAAACCCATTTGGAGAGTGCAAAATGAA

AAACAAAAACAAAAGTAGAAAGCAAGAGGGAACTTTCAGAAAATGGAAACCATGGGCTCCTATTTAACACACAGGCCTG

AAGGAAGGTCTTCAGAGAACCTAGAAAGCAGGTTCACAGAGTCACCCACCTCCCCAGGCCACAAGCATCTGCAAGGTCC

```
      S1                                           S10
      met ala pro ala trp ser leu leu leu ala leu leu leu leu ser cys asn ala ile
      CCA ATG GCC CCA GCC TGG TCC TTA CTG CTG GCC CTG CTG CTG CTC AGC TGC AAT GCC ATC

S20        S23  1                                  10
cys ser leu gly cys his leu pro his thr his ile leu ala asn arg arg val leu met
TGC TCT CTG GGC TGC CAC CTG CCT CAC ACC CAC ATC CTG GCC AAC AGG AGG GTC CTG ATG

             20                              30
leu leu gly gln leu arg arg val ser pro ser ser cys leu gln asp arg asn asp phe
CTC CTG GGA CAA CTG AGG AGG GTC TCC CCT TCC TCC TGC CTG CAG GAC AGA AAT GAC TTT

             40                              50
ala phe pro gln glu ala leu gly gly ser gln leu gln lys ala gln ala ile ser val
GCA TTC CCC CAG GAG GCG CTG GGT GGC AGC CAG TTG CAG AAG GCT CAA GCC ATC TCT GTG

             60                              70
leu his glu val thr gln his thr phe gln leu phe ser thr glu gly ser ala thr met
CTC CAC GAG GTG ACC CAG CAC ACC TTC CAG CTT TTC AGC ACA GAG GGC TCG GCC ACC ATG

             80                              90
trp asp glu ser leu leu asp lys leu arg asp ala leu asp gln gln leu thr asp leu
TGG GAT GAG AGC CTC CTG GAC AAG CTC CGC GAT GCA CTG GAT CAG CAG CTC ACT GAC CTG

             100                             110
gln phe cys leu arg gln glu glu glu leu gln gly ala pro leu leu lys glu asp ser
CAA TTC TGT CTG AGG CAG GAG GAG GAG CTG CAA GGA GCT CCC CTG CTC AAG GAG GAC TCC

             120                             130
ser leu ala val arg lys tyr phe his arg leu thr leu tyr leu gln glu lys arg his
AGC CTG GCT GTG AGG AAA TAC TTC CAC AGA CTC ACT CTC TAT CTG CAA GAG AAG AGA CAC

             140                             150
ser pro cys ala trp glu val val arg ala gln val met arg ala phe ser ser ser thr
AGC CCT TGT GCC TGG GAG GTT GTC AGA GCA CAA GTC ATG AGA GCC TTC TCT TCC TCA ACA

             160             166
asn leu gln glu ser phe arg arg lys asp OP
AAC TTG CAG GAG AGT TTC AGG AGA AAG GAC TGA CACACACCTGGTTCAACACGGAAATGATTCTCATGG
```

ACCAACAGACCACACTTCCTCCTGCGCTGCCATGTGGAAGATTCATTTCTGCTGTCATCAGGCACTGAACTGAATCAA

TTTGTTAAATGATTTCAGGTATATTAAGCGACATCATGATCTACTCTACAGGCACTACTCTGTCCCGGATACTCAAGC

TAATCCATCTACTTATTTATCTATTTGGATATTTATTTATCTAATTTAATATTTATTTATCTATATATAAAGAATTAA

ATTATTTTGTTCATATAATTATGTATGTATACTTAAGGGAAAAAATATATTTTGTATTTAGTCAATTTATGAGTTTTCT

TCATTCATTAAACCTTACTATAAAAATCTTCCTTTGTTTTTCTTTAAAAAAGAAACATGAAGACTGAATACGCAACTT

GATTAAAGAATGCATTTTACAATTCCTTCACCCATGTTTGTGATTTCCACATTACAAGTGTTGGGGATTTTCTCCTCG

GGACTTGGAAGCGACGAACCTGAAAGAAGGACGGACAGTCTCTTGCAAGGACTGAC

*Fig.3c*

BoIFN-α4

TCTAGAAGGAAAAGAAACATGGGAGGAGCTCATGGACTCACAATAATCAGCCTCTTCTCTAAATCAAACACTCAAGAAA

ACCCATCCTTTGTTTCAAACAGTTTCTTTGCTAGAGTGACCTGGTAAATGCCTGATAAACACAGAGCCTGCCTTCCTAA

GTAAAGTAAGAAACAGAAATAAGCCTAGGATCAGGGCCAAAAATGTGTTCATGAACAGAAAAGAGCCACATTTACACAT

GAAGAGAAAAGTGATATGTTTGTACTTAGAAACCTACATCATTTCTAATGTAAGACAAAGACCTTTCTCATTTGATTGA

TAAATATCCATTTGGATGGGTACATTTGAAGTTATTGGGAAATAGATAAAAGTAATAGTTTAGACTGATGACAATTTCT

TTGACCATATTCGGAATACATAAATGAAAATCAAAAAAGGAAGTACAAGTACACAGAAATGACTAGAAAATGAAAATTA

CTGTGTTCCCTATTTAATGGCCTTGCTTAGAAAGCATGGCATCAGAGAACCTACCTCAAGGTTCCACCAGACGCTGTCT

```
                                    S1                                    S10
                             met ala phe val leu ser leu leu met ala leu val
CAGCCAGCCCAGCAGCAGCCTCATCTTCCCC ATG GCC TTC GTG CTC TCT CTA CTG ATG GCC CTG GTG

              S20           S23   1
leu val ser tyr gly pro gly gly ser leu gly cys asp leu ser pro asn his val leu
CTG GTC AGC TAT GGC CCG GGA GGA TCC CTG GGC TGT GAC TTG TCT CCG AAC CAC GTG CTG

 10                              20
val gly arg gln asn leu arg leu leu gly gln met arg arg leu ser pro arg phe cys
GTT GGC AGG CAG AAC CTC AGG CTC CTG GGC CAA ATG AGG AGA CTC TCC CCT CGC TTC TGT

 30                      40
leu gln asp arg lys asp phe ala phe pro gln glu met val glu val ser gln phe gln
CTG CAG GAC AGA AAA GAC TTT GCT TTC CCC CAG GAG ATG GTG GAG GTC AGC CAG TTC CAG

 50                              60
glu ala gln ala ile ser val leu his glu met leu gln gln ser phe asn leu phe his
GAG GCC CAG GCC ATT TCT GTG CTC CAT GAG ATG CTC CAG CAG AGC TTC AAC CTC TTC CAC

 70                              80
lys glu arg ser ser ala ala trp asp thr thr leu leu glu gln leu leu thr gly leu
AAA GAG CGC TCC TCT GCT GCC TGG GAC ACT ACC CTC CTG GAG CAG CTC CTC ACT GGA CTC

 90                              100
his gln gln leu asp asp leu asp ala cys leu gly leu leu thr gly glu glu asp ser
CAT CAG CAG CTG GAT GAC CTG GAT GCC TGT CTG GGC CTG TTG ACT GGA GAG GAA GAC TCT

110                              120
ala leu gly arg thr gly pro thr leu ala met lys arg tyr phe gln gly ile his val
GCC CTG GGA AGG ACG GGC CCC ACA CTG GCC ATG AAG AGG TAC TTC CAG GGC ATC CAT GTC

130                              140
tyr leu gln glu lys gly tyr ser asp cys ala trp glu ile val arg leu glu ile met
TAC CTG CAA GAG AAG GGA TAC AGC GAC TGT GCC TGG GAA ATC GTC AGA CTG GAA ATC ATG

150                              160
arg ser leu ser ser ser thr ser leu gln glu arg leu arg met met asp gly asp leu
AGA TCC TTG TCT TCA TCA ACC AGC TTG CAA GAA AGG TTA AGA ATG ATG GAT GGA GAC CTG

170     172
lys ser pro OP
AAA TCA CCT TGA CATGACTCTCACTGACTAAGATGCCCCATCATCTTTGCACACTCATCTGTGGCCATTTCAAAA
```

GACTCTGATTTCTGTTGTAGCCACAAAATTTATTGAATTACTTCAGCCAATACTTTGTCAGTAGTAAATGAATATACA

TAAATTTTTTTGGCTGCAGGTGCATCAGTCCCGAAGTGAAGACTGCCCTTATTTTATTGTTTGCTTATTTATTTTGTT

ATATTTATTCTTTTATTTCCTCATATTTATTTTTTCCATATAAAATATTTTTGTTTACATTGTATTAAAAATTTAACAAA

TACATTAACATTTTTATTTCATTATATTTGTAATTTGTTTTATTTATTAAATATTGTCAAGGTGAACTTCTTGAATTT

TTTTACCGTTTTATGTTTAGTTGCCAGGTAAAGTCTGATTCTTTTGTGACCCCATAGACTGTAGCCCACCAGGCTCCT

CTGTCCATGGGATGATCAGGCAAGACTACTGGAGTATGTTGCCATTTACAGGGGATCTCCCAACCAAGGATGAAATC

## Fig.3d

Fig. 4a

```
                    50              60              70              80              90             100
                    ·               ·               ·               ·               ·              ·
BoIFN-α1   E A L G G S Q L Q K A Q A I S V L H E V T Q H T F Q L F S T E G S A A V W D E S L L D R L R T A L D Q Q L T D L Q A C L R Q E
BoIFN-α2   E A L G G S Q L Q K A Q A I S V L H E V T Q H T F Q L F S T E G S A A V W D Q S L L D K L R A A L D Q Q L T D L Q A C L R Q E
BoIFN-α3   E A L G G S Q L Q K A Q A I S V L H E V T Q H T F Q L F S T E G S A T M W D E S L L D K L R D A L D Q Q L T D L Q F C L R Q E
BoIFN-α4   E M V E V S Q F Q E A Q A I S V L H E M L Q Q S F N L F H K E R S S A A W D T T L L E Q L L L T G L H Q Q L D D L D A C L G L L
LeIF A     E E F - G N Q F Q K A E T I P V L H E M I Q Q I F N L F S T K D S S A A W D E T L L D K F Y T E L Y Q Q L N D L E A C V I Q G
LeIF B     E E F D D K Q F Q K A Q A I S V L H E M I Q Q T F N L F S T K D S S A A L D E T L L D E F Y I E L D Q Q L N D L E V L C D Q E
LeIF C     E E F D G N Q F Q K A Q A I S V L H E M I Q Q T F N L F S T E D S S A A W E Q S L L E K F S T E L Y Q Q L N D L E A C V I Q E
LeIF D     E E F D G N Q F Q K A P A I S V L H E L I Q Q I F N L F T T K D S S A A W D E D L L D K F C T E L Y Q Q L N D L E A C V M Q E
LeIF E     Q V F H G N H F Q K V Q A I F L F H E M M Q Q T F N L F S T K D S S D T W D E T L L D K S Y T E L Y Q Q L N D L E A C V M * K
LeIF F     E E F D G N Q F Q K A Q A I S V L H E M I Q Q T F N L F S T K D S S A T W E Q S L L E K F S T E L N Q Q L N D M E A C V I Q E
LeIF G     E E F D G N Q F Q K A Q A I S V L H E M I Q Q T F N L F S T K D S S A T W D E T L L D K F Y T E L Y Q Q L N D L E A C M M Q E
LeIF H     E E F D G N Q F Q K A Q A I S V L H E M M Q Q T F N L F S T K N S S A A W E Q S L L E K F Y I E L F Q Q M N D L E A C V I Q E
LeIF I     E E F D G N Q F Q K T Q A I S V L H E M I Q Q T F N L F S T E D S S A A W E Q S L L E K F S T E L Y Q Q L N N L E A C V I Q E
LeIF J     E E F D G H Q F Q K T Q A I S V L H E M I Q Q T F N L F S T E D S S A A W E Q S L L E K F S T E L Y Q Q L N D L E A C V I Q E
LeIF K     E E F D G N Q F Q K A E A I S V L H E V I Q Q T F N L F S T K D S S V A W D E R L L D K L Y T E L·Y Q Q L N D L E A C V M Q E

All Human      E E F       Q F Q K       I   V L H E     Q Q   F N L F   T     S S             L L         E L   Q Q   N     E           Q
All Bovine     E       S Q   Q   A Q A I S V L H E     Q     F   L F   E   S         D     L L       L     L   Q Q L   D L     C L     Q
BoIFN-α1/Human E       G   Q   Q K A Q A I S V L H E     Q   T F   L F S T     S   A   W D E S L L D       T   L   Q Q L   D L   A C       Q E
BoIFN-α4/Human E           Q F Q   A Q A I S V L H E M   Q Q   F N L F         S S A A W D   T L L E       T   L   Q Q L   D L   A C
```

# Fig.4b

EP 0 088 622 B1

```
              110            120            130            140            150            160      166
              .              .              .              .              .              .        .
BoIFN-α1   E G L P G A P L L K E D S S L A V R K Y F H R L T L Y L Q E K R H S P C A W E V V R A Q V M R A F S S S T N L Q E R F R R K D
BoIFN-α2   E G L R G A P L L K E D A S L A V R K Y F H R L T L Y L Q E K R H S P C A W E V V R A E V M R A F S S S T N L Q E K F R R K D
BoIFN-α3   E E L Q G A P L L K E D S S L A V R K Y F H R L T L Y L Q E K R H S P C A W E V V R A Q V M R A F S S S T N L Q E S F R R K D
BoIFN-α4   T G E E D S A L G R T G P T L A M K R Y F Q G I H V Y L Q E K G Y S D C A W E I V R L E I M R S L S S S T S L Q E R L R M M D G D L K S P
LeIF A     V G V T E T P L M K E D S I L A V R K Y F Q R I T L Y L K E K K Y S P C A W E V V R A E I M R S F S L S T N L Q E S L R S K E
LeIF B     V G V I E S P L M Y E D S I L A V R K Y F Q R I T L Y L T E K K Y S S C A W E V V R A E I M R S F S L S I N L Q K R L K S K E
LeIF C     V G V E E T P L M N E D S I L A V R K Y F Q R I T L Y L I E R K Y S P C A W E V V R A E I M R S L S F S T N L Q K R L R R K D
LeIF D     E R V G E T P L M N V D S I L A V K K Y F R R I T L Y L T E K K Y S P C A W E V V R A E I M R S L S L S T N L Q E R L R R K E
LeIF E     V G V E E T P L R N V D S I L A V R K Y F Q R I T L Y L T K K K Y S P C S W E A V R A E I M R S F S L * T N L Q E R L R R K E
LeIF F     V G V E E T P L M N V D S I L A V K K Y F Q R I T L Y L T E K K Y S P C A W E V V R A E I M R S F S L S K I F Q E R L R R K E
LeIF G     V G V E D T P L M N V D S I L T V R K Y F Q R I T L Y L T E K K Y S P C A W E V V R A E I M R S F S L S A N L Q E R L R R K E
LeIF H     V G V E E T P L M N E D S I L A V R K Y F Q R I T L Y L M E K K Y S P C A W E V V R A E I M R S F S F S T N L Q K R L R R K D
LeIF I     V G M E E T P L M N E D S I L A V R K Y F Q R I T L Y L T E K K Y S P C A W E V V R A E I M R S L S F S T N L Q K I L R R K D
LeIF J     V G V K E T P L M N E D F I L A V R K Y F Q R I T L Y L M E K K Y S P C A W E V V R A E I M R S F S F S T N L K K G L R R K D
LeIF K     V W V G G T P L M N E D S I L A V R K Y F Q R I T L Y L T E K K Y S P C A W E V V R A E I M R S F S S S R N L Q E R L R R K E

All Human           P L M     D   I L   V   K Y F   R I T L Y L   E   K Y S   C A W E V V R A E I M R S   S S                   L   K
All Bovine   E            L             L A       Y F         Y L Q E K     S   C A W E V V R     M R       S S S T   L Q E     R
BoIFN-α1/Human      P L   E D S   L A V R K Y F   R   T L Y L   E K     S P C A W E V V R A     M R   F S   S T N L Q E R     R R K D
BoIFN-α4/Human   G   E      L             L A       Y F Q   I   Y L   E K   Y S   C A W E   V R   E I M R S L S   S T   L Q E R L R       D
```

Fig. 4c

Fig.5

*Fig.6*

kilobases

15       10       5       0

λβ1

5' 3'

Bam HI

Hind III

Bgl II*

Xho I

Bgl II*

Bam HI

R—arm

λβ2

3' 5'

Bgl II*    Bam HI

Bgl II*    Bam HI

Bam HI

Hind III

Xho I

Hind III    Hind III

Bgl II*

R—arm

λβ3

Bam HI

Bam HI

Eco RI*   Bam HI

3' 5'

Sal I

Hind III   Hind III

Sal I

Xho I

Bgl II*

R—arm

Fig.7

Fig.8

EP 0 088 622 B1

BoIFN-β1

AGTTTTAGAGGGCAACTAATAATTTAATGACATGGAAAAATGAAAGCGAGAACTGAAAGTGGGAAATTCCTCTGAAATA

GAAAGATGAGGGCCATGCTGTATAAGTAGCCCACACTAAGGACGAGGACATTCACTCTGCAAACCCTTGAAGACTCAGC

```
                                                              S1
                                                              met thr tyr arg cys leu leu gln
TTCAGCACCTACTAGCAGAACAGGTAGCCCTGTGCCTGATTTCATC  ATG ACC TAC CGG TGC CTC CTC CAG

      S10                                            S20       1
met val leu leu leu cys phe ser thr thr ala leu ser arg ser tyr ser leu leu arg
ATG GTT CTC CTG CTG TGT TTC TCC ACC ACA GCT CTT TCC AGG AGC TAC AGC TTG CTT CGA

          10                                    20
phe gln gln arg gln ser leu lys glu cys gln lys leu leu gly gln leu pro ser thr
TTC CAA CAA CGT CAG AGC CTT AAA GAG TGT CAG AAA CTC CTG GGG CAG TTA CCT TCA ACT

          30                                    40
ser gln his cys leu glu ala arg met asp phe gln met pro glu glu met lys gln glu
TCT CAA CAT TGC CTC GAG GCC AGG ATG GAC TTC CAG ATG CCT GAG GAG ATG AAG CAA GAA

          50                                    60
gln gln phe gln lys glu asp ala ile leu val met tyr glu val leu gln his ile phe
CAG CAG TTC CAG AAG GAA GAT GCC ATA TTG GTC ATG TAT GAG GTG CTC CAG CAC ATC TTC

          70                                    80
gly ile leu thr arg asp phe ser ser thr gly trp ser glu thr ile ile glu asp leu
GGC ATT CTC ACC AGA GAC TTC TCC AGC ACT GGC TGG TCT GAG ACC ATC ATC GAG GAC CTC

          90                                    100
leu lys glu leu tyr trp gln met asn arg leu gln pro ile gln lys glu ile met gln
CTT AAG GAA CTC TAT TGG CAG ATG AAT CGT CTG CAG CCA ATC CAG AAG GAA ATA ATG CAG

          110                                   120
lys gln asn ser thr thr glu asp thr ile val pro his leu gly lys tyr tyr phe asn
AAG CAA AAC TCC ACT ACG GAA GAC ACG ATC GTT CCC CAC CTA GGG AAA TAT TAC TTC AAC

          130                                   140
leu met gln tyr leu glu ser lys glu tyr asp arg cys ala trp thr val val gln val
CTC ATG CAG TAC CTG GAG TCC AAG GAG TAC GAC AGG TGT GCC TGG ACA GTC GTG CAA GTG

          150                                   160          165
gln ile leu thr asn val ser phe leu met arg leu thr gly tyr val arg asp OP
CAA ATA CTC ACG AAC GTT TCT TTC CTG ATG AGA CTA ACA GGT TAC GTC CGT GAC TGA ACA
```

TCTCCCACCTGTGGCTCTGGGAAGGGACAATGTGACTTTGAGGTGAGACTCTTCACCCAGCAGAGGCTCTTGAAGTAA

CTGACAATGCAATGCACTGGATTTCAATGGACAGTTAAGACTAAGCTATTTTAAATTGATTTATGCATTATTTATTTA

TTTATTTAATGAGAAATAAATTATTTATCAAAATTTATTTAATGAGAAATAAATTATTTATGAAACAAAAGTCAACGT

GGCAGTTTCAATCTCAACTTGATTTATGTGACAACACATATTAAAAAATTGCAGAGCACTTTGGAGACATTCATTGCAA

AACAAGCCTGCAGAGTAGTAGAGTTTCTGGCCCTGCCTTTGAGGCATTTAAAATACAAGGAAGCTGTTTGGAATGTCC

AAGTTATATGCATGCTCTCCATGT

## Fig.9a

43

BoIFN-β2


TTTTAGCATTTAGCAATTCACTGAAAAATTTACAAAAACATTAGAAATTCTCCCAGACTGTATATCTTTTTCCCCTTAAT

ACATATAAAATCAAAAAGCAAGGAGCTAAAAAGAAAAAGAGTTTTAGAGGTAACTAATCAACACAGGAGAACTAAAAAG

GAAACTGGAAAGTGGGAAATTCCTCTCCAATAGAAAGAATGGAGGGCCATGCTGTATAAGTAGCCCACACTCAAGAAGG

AAGGCCATTCACTCTGCAAACCCTTGAAGACTCAGCTTCAGCACCTACTAGCAGAACAGGCAGCCCTGTGCCTGATTTC

```
       S1                                      S10
       met thr his arg cys leu leu gln met val leu leu leu cys phe ser thr thr ala
       ATC ATG ACC CAC CGG TGC CTC CTC CAG ATG GTT CTC CTG CTG TGT TTC TCC ACC ACA GCT

       S20       1                                  10
       leu ser arg ser tyr ser leu leu arg phe gln gln arg arg ser leu glu leu cys gln
       CTT TCC AGG AGC TAC AGC TTG CTT CGA TTC CAA CAA AGG CGG AGC CTT GAG TTA TGT CAG

          20                                  30
       lys leu leu arg gln leu pro ser thr pro gln his cys leu glu ala lys met asp phe
       AAA CTC CTG AGG CAG TTA CCT TCA ACT CCT CAA CAT TGC CTC GAG GCC AAG ATG GAC TTC

          40                                  50
       arg met pro glu glu met lys gln ala gln gln phe arg lys glu asp ala ile leu val
       CGG ATG CCT GAG GAG ATG AAG CAA GCA CAG CAG TTC CGG AAG GAA GAT GCC ATA TTG GTC

          60                                  70
       ile tyr glu met leu gln gln ile phe asn ile leu thr arg asp phe ser ser thr gly
       ATC TAT GAG ATG CTC CAG CAG ATC TTC AAT ATT CTC ACC AGA GAC TTC TCC AGC ACT GGC

          80                                  90
       trp ser glu thr ile ile glu asp leu leu glu glu leu tyr glu gln met asn his leu
       TGG TCT GAG ACC ATC ATC GAG GAC CTC CTT GAG GAA CTC TAT GAG CAG ATG AAT CAT CTG

          100                                 110
       glu pro ile gln lys glu ile met gln lys gln asn ser thr met gly asp thr thr val
       GAG CCA ATC CAG AAG GAA ATA ATG CAG AAG CAA AAC TCC ACT ATG GGA GAC ACA ACC GTT

          120                                 130
       leu his leu arg lys tyr tyr phe asn leu val gln tyr leu lys ser lys glu tyr asn
       CTT CAC CTG AGG AAA TAT TAC TTC AAC CTC GTG CAG TAC CTA AAG TCC AAG GAG TAC AAC

          140                                 150
       arg cys ala trp thr val val arg val gln ile leu arg asn phe ser phe leu thr arg
       AGG TGT GCC TGG ACA GTC GTG CGA GTG CAA ATA CTC AGG AAT TTT TCT TTC CTG ACG AGA

          160           165
       leu thr gly tyr leu arg glu OP
       CTA ACA GGT TAC CTC CGT GAA TGA ACATCTCCCACCTGTGGCTCTGGGATTGACAATGTGACTTTGAGGTGA
```

GACTCTTCACCCAGTAGAGGCTCTTGAAGTAACTGACAATGCAATGCAATGGACAGTTAAATACTGTAAGCTATTTTT

AAATTGATTTATGCATTATTTATTTATTTAAACTTTTATATGGGAAATAAATTATTTATGAAACAAAATTGAACATGG

CAGTTTTAATGTCAACTTGATTGATGTGACAACATATATTAAAAAATTGGCGAGCACCCTGGAGACATTTATTGCAAAA


# Fig.9b

BoIFN-β3


TTGCATAAGAGGCTATTTAGTCCTCTTCTACTTTCTGCCAAAGTTATAGAGGCAACGAATAATTTAAATGACAAAGGAA

AACTGAAAGGGAGAACTGAAAGTGGGAAATCTCTCCGAGGGCCATCCTATATAAGTAGCCCACACTCAAGGAGGAAGGC
CATTCACTCTGCAAGCCCTTGAAGACTCAGCGTCAGCATCTACTAGCAGAACGGGCAGCCCTGTGCCTGTTTTCATC

```
 S1                                        S10                                  S20
 met thr tyr arg cys leu leu pro met val leu leu leu cys phe ser thr thr ala leu
 ATG ACC TAC CGG TGC CTC CTC CCG ATG GTT CTC CTG CTG TGT TTC TCC ACC ACA GCT CTT

      1                                    10
 ser arg ser tyr ser leu leu arg phe gln gln arg arg ser ala glu val cys gln lys
 TCC AGG AGC TAC AGC TTG CTC AGA TTC CAG CAA AGG CGG AGC GCT GAG GTG TGT CAG AAA

  20                                      30
 leu leu gly gln leu his ser thr pro gln his cys leu glu ala lys met asp phe gln
 CTC CTG GGG CAG TTA CAT TCA ACG CCT CAA CAT TGC CTC GAG GCC AAG ATG GAC TTC CAA

  40                                      50
 val pro glu glu met asn gln ala gln gln phe arg lys glu asp ala ile leu val ile
 GTC CCT GAG GAG ATG AAC CAA GCA CAG CAG TTC CGG AAG GAA GAT GCC ATA TTG GTC ATC

  60                                      70
 tyr glu met leu gln gln ile phe asn ile leu thr arg asp phe ser ser thr gly trp
 TAT GAG ATG CTC CAG CAG ATC TTC AAT ATT CTC ACC AGA GAC TTC TCC AGC ACT GGC TGG

  80                                      90
 ser glu thr ile ile glu asp leu leu val glu leu tyr gly gln met asn arg leu gln
 TCT GAG ACC ATC ATT GAG GAC CTC CTT GTG GAA CTC TAT GGG CAG ATG AAT CGT CTG CAG

 100                                     110
 pro ile gln lys glu ile met gln glu gln asn phe thr met gly asp thr thr val leu
 CCA ATC CAG AAG GAA ATA ATG CAG GAG CAA AAC TTC ACC ATG GGG GAC ACA ACC GTT CTT

 120                                     130
 his leu lys lys tyr tyr phe asn leu val gln tyr leu glu ser lys glu tyr asn arg
 CAC CTG AAG AAG TAT TAC TTC AAC CTC GTG CAG TAC CTG GAG TCC AAG GAG TAC AAC AGG

 140                                     150
 cys ala trp thr val val arg val gln ile leu thr asn phe ser phe leu met arg leu
 TGT GCC TGG ACA GTC GTG CGA GTG CAA ATA CTC ACG AAC TTT TCT TTC CTG ATG AGA CTA

 160             165
 thr ala ser leu arg asp OP
 ACA GCT TCC CTC CGT GAC TGA ACACCTCCCACCTGTGGCTCTGGGAAGGGACAATGTGACTTTGAGCTGAGA
```

TGCTTCAGCCAGCAGAGGCTCTTAAAGTAACTGACAGTGCAATGCACGATTTCAATGAACAATAAAGACTAAGCTATT

TTTAAATTGATTTATGCGTTATTTCATTCATTTAAACTTTTATGTGAGAAATAAAT


# Fig.9c

EP 0 088 622 B1

```
          S1                S10              S20  1                10               20               30               40
BoIFN-β1  M T Y R C L L Q M V L L L C F S T T A L S R S Y S L L R F Q Q R Q S L K E C Q K L L G Q L P S T S Q H C L E A R M D F Q M P E
BoIFN-β2  M T H R C L L Q M V L L L C F S T T A L S R S Y S L L R F Q Q R R S L E L C Q K L L R Q L P S T P Q H C L E A K M D F R M P E
BoIFN-β3  M T Y R C L L P M V L L L C F S T T A L S R S Y S L L R F Q Q R R S A E V C Q K L L G Q L H S T P Q H C L E A K M D F Q V P E
HuIFN-β   M T N K C L L Q I A L L L C F S T T A L S M S Y N L L G F L Q R S S N F Q C Q K L L W Q L N G R L E Y C L K D R M N F D I P E

                    50               60               70               80               90               100
BoIFN-β1  E M K Q E Q Q F Q K E D A I L V M Y E V L Q H I F G I L T R D F S S T G W S E T I I E D L L K E L Y W Q M N R L Q P I Q K E I
BoIFN-β2  E M K Q A Q Q F R K E D A I L V I Y E M L Q Q I F N I L T R D F S S T G W S E T I I E D L L E E L Y E Q M N H L E P I Q K E I
BoIFN-β3  E M N Q A Q Q F R K E D A I L V I Y E M L Q Q I F N I L T R D F S S T G W S E T I I E D L L V E L Y G Q M N R L Q P I Q K E I
HuIFN-β   E I K Q L Q Q F Q K E D A A L T I Y E M L Q N I F A I F R Q D S S S T G W N E T I V E N L L A N V Y H Q I N H L K T V L E E K

                    110              120              130              140              150              160
BoIFN-β1  M Q K Q N S T T E D T I V - P H L G K Y Y F N L M Q Y L E S K E Y D R C A W T V V Q V Q I L T N V S F L M R L T G Y V R D
BoIFN-β2  M Q K Q N S T M G D T T V - L H L R K Y Y F N L V Q Y L K S K E Y N R C A W T V V R V Q I L R N F S F L T R L T G Y L R E
BoIFN-β3  M Q E Q N F T M G D T T V - L H L K K Y Y F N L V Q Y L E S K E Y N R C A W T V V R V Q I L T N F S F L M R L T A S L R D
HuIFN-β   L E K E D F T R G K L M S S L H L K R Y Y G R I L H Y L K A K E Y S H C A W T I V R V E I L R N F Y F I N R L T G Y L R N
```

## Fig.10

Fig.11

Fig.12

EP 0 088 622 B1

```
          S1                 S10                 S20 1                 10                  20                  30
          |                   |                   |  |                  |                   |                   |
HuIFN-γ   M K Y T S Y I L A F Q L C I V L G S L G C Y C Q D P Y V K E A E N L K K Y F N A G H S D V A D N G T L F L G I
MuIFN-γ   M N A T H C I L A L Q L F L M A V S G - C Y C H G T V I E S L E S L N N Y F N S S G I D V E E K - S L F L D I
BoIFN-γ   M K Y T S Y F L A L L L C G L L G F S G S Y G Q G Q F F R E I E N L K E Y F N A S S P D V A K G G P L F S D I
consensus M - - T - - - L A - - - - - - - - - - - - Y - - - - - - - - - E - L - - Y F N - - - - D V - - - - - L F - - I


                     40                  50                  60                  70                  80                  90
                      |                   |                   |                   |                   |                   |
HuIFN-γ   L K N W K E E S D R K I M Q S Q I V S F Y F K L F K N F K D D Q S I Q K S V E T I K E D M N V K F F N S N K K
MuIFN-γ   W R N W Q K D G D M K I L Q S Q I I S F Y L R L F E V L K D N Q A I S N N I S V I E S H L I T T F F S N S K A
BoIFN-γ   L K N W K D E S D K K I I Q S Q I V S F Y F K L F E N L K D N Q V I Q R S M D I I K Q D M F Q K F L N G S S E
consensus - - N W - - - - D - K I - Q S Q I - S F Y - - L F - - - K D - Q - I - - - - - - I - - - - - - - - F - - - - - -


                     100                 110                 120                 130                 140             146
                      |                   |                   |                   |                   |               |
HuIFN-γ   K R D D F E K L T N Y S V T D L N V Q R K A I H E L I Q V M A E L S P A A K T G K R K R S Q M L F R - G R R A S Q
MuIFN-γ   K K D A F M S I A K F E V N N P Q V Q R Q A F N E L I R V V H Q L L P E S S L R K R K R S R C W F G V G K R L S Q
BoIFN-γ   K L E D F K K L I Q I P V D D L Q I Q R K A I N E L I K V M N D L S P K S N L R K R K R S Q N L F R - G R R A S M
consensus K - - - F - - - - - - - - V - - - - - Q R - A - - E L I - V - - - L - P - - - - - K R K R S - - - F - - G - R - S -
```

*Fig. 13*